(19)

Europäisches
Patentamt
European
Patent Office
Office européen
des brevets

(11) **EP 4 400 117 A1**

(12) **EUROPEAN PATENT APPLICATION**
published in accordance with Art. 153(4) EPC

(43) Date of publication:
**17.07.2024  Bulletin 2024/29**

(21) Application number: **22867349.7**

(22) Date of filing: **06.09.2022**

(51) International Patent Classification (IPC):
**A61K 45/00** *(2006.01)*

(52) Cooperative Patent Classification (CPC):
**A61K 31/08; A61K 31/138; A61K 31/166;**
**A61K 31/352; A61K 31/366; A61K 31/4025;**
**A61K 31/403; A61K 31/407; A61K 31/409;**
**A61K 31/4155; A61K 31/4178; A61K 31/4188;**
**A61K 31/4196; A61K 31/437; A61K 31/4375;**

(Cont.)

(86) International application number:
**PCT/JP2022/033445**

(87) International publication number:
**WO 2023/038027 (16.03.2023 Gazette 2023/11)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority:  **07.09.2021   JP 2021145812**

(71) Applicant: **Kyoto University**
**Kyoto-shi, Kyoto 606-8501 (JP)**

(72) Inventors:
• **KONDOH Hiroshi**
  **Kyoto-shi, Kyoto 606-8501 (JP)**
• **MIKAWA Takumi**
  **Kyoto-shi, Kyoto 606-8501 (JP)**

(74) Representative: **Mewburn Ellis LLP**
**Aurora Building**
**Counterslip**
**Bristol BS1 6BX (GB)**

Remarks:
The complete document including Reference
Table(s) and the Sequence Listing(s) can be
downloaded from the EPO website

(54) **SENOLYTIC DRUG SCREENING METHOD AND SENOLYTIC DRUG**

(57)    The purpose of the present invention is to provide a screening method that enables efficient discovery of a novel senolytic drug and a senolytic drug obtained by the screening method. The present invention pertains to a senolytic drug that inhibits binding between PGAM and Chkl and also selectively kills senescent cells. More particularly, the senolytic drug of the present invention contains, as an active ingredient, at least one selected from the group consisting of an mRSK1 kinase inhibitor, an Fak kinase inhibitor, an inhibitor of signaling pathways involving Fak kinase, a CDK inhibitor, a calcium antagonist agent, an inotropic glycoside, a DNA damaging agent, an antimicrobial agent, an Aurora kinase inhibitor, a flavonoid, a PI3K kinase inhibitor, an HDAC inhibitor, a therapeutic agent for age-related macular degeneration, a p38 MAPK inhibitor, an mTOR inhibitor, a tyrosine kinase inhibitor, a Bcl-2 inhibitor, statin, a serotonin receptor antagonist, other kinase inhibitors and Nutlin-3b.

**(Cont. next page)**

EP 4 400 117 A1

Fig. 29

(52) Cooperative Patent Classification (CPC): (Cont.)
A61K 31/438; A61K 31/444; A61K 31/4709;
A61K 31/473; A61K 31/495; A61K 31/498;
A61K 31/4985; A61K 31/502; A61K 31/505;
A61K 31/506; A61K 31/519; A61K 31/52;
A61K 31/53; A61K 31/7012; A61K 31/7028;
A61K 31/704; A61K 31/7088; A61K 31/713;
A61K 45/00; A61P 43/00; C12Q 1/02;
G01N 33/15; G01N 33/52

**Description**

TECHNICAL FIELD

The present invention relates to a method for screening senolytic drugs and novel senolytic drugs obtained by the screening method.

BACKGROUND ART

Currently, Japan is the only country in the world having a population aging rate exceeding 25% (2014). The world average of the population aging rate is 8%, but the wave of global population aging is steadily sweeping the world, and it is forecasted that the population aging rate will exceed 25% in European countries, China, South Korea, Thailand, Singapore, Iran, Chile, Canada, and other countries around 2050, and that the world average thereof will also be 18% (WHO forecast). Here, the reality of global population aging is health promotion in elderly people in general and simultaneously an increase in the number of multi-morbid and diverse elderly people. Therefore, the definition of "aging" based only on the time axis does not fit to the current situation, and the redefinition of elderly people is necessary (2017 proposal by the Japan Geriatrics Society); and the development of a pharmaceutical based on an innovative approach to the increasing number of age-related diseases is becoming an urgent issue.

The gap between the "chronological age" and the current situation (symptoms) of elderly people has also been pointed out according to the recent findings on cellular senescence in basic aging research. As cells age, the length of a telomere (chromosomal end structure that shortens with each cell division) shortens, and thus conventionally, the telomere has also been called an "aging clock." However, in recent years, "stress-induced senescence" (even young cells, that is, cells having long telomeres age because of various stresses) was found as telomere-independent cellular senescence. While this "stress-induced senescence" has been attracting attention as a "biological defense mechanism *in vivo* for suppressing carcinogenesis" (beneficial effect), it has been found that stress-induced senescent cells affect surrounding cells and also contribute to a harmful effect such as "chronic inflammation" or "canceration." This finding is probably one of the most prominent achievements in recent basic aging research.

Specifically, first, a detailed study by Adler et al. revealed that one of the reasons why "senescent cells are less likely to die than young cells" is the acquisition of an anti-apoptotic ability (resistance to cell death) such as Bcl-2 activation (Non Patent Document 1: Adler et al., 2007). The transcription factor responsible for the Bcl-2 activation is NF-κB. NF-κB is also a key factor for controlling inflammation and also causes activation of inflammation in senescent cells. At almost the same time, Campisi et al. found that senescent cells are more likely to secrete an inflammatory cytokines such as IL-1 or IL-6 than young cells, which Campisi et al. termed SASP (senescence associated secretary phenotype) and reported (Non Patent Document 2: Coppe et al., 2008). The senescent cell-derived SASP is thought to contribute to the provocation of senescence or the promotion of canceration in surrounding cells, and cellular senescence is thought to be one of the factors driving tissue or organismal aging through "chronic inflammation." Based on the above findings, "stress-induced senescence" has been assumed to be one of the major causes of the progress of aging or age-related diseases.

Based on the above findings, "senolysis" (removal of senescent cells) has emerged as a possibility for novel medical development for age-related diseases. As described above, this is being proposed based on the innovative idea that organismal aging can be improved by eliminating senescent cells, which are a major source of chronic inflammation. Darren Baker et al. successfully achieved senolysis in which only senescent cells (cells positive for p 16INK4a, which is a senescence marker) is selectively killed in a BubR1 hypomorphic mutant mouse, a model mouse that exhibits premature aging, and at the same time also improved the aging phenotypes of various organs in this mutant mouse. This was the first report of alleviating aging phenotype in an mouse model by using genetical method for senolysis (Non Patent Document 3: Baker et al., 2011). In addition, recently, an agent (ABT263) that exerts a similar beneficial effect was found and has been called as a senolytic drug (Non Patent Document 4: Chang et al., 2016). Conventionally, ABT263 is an anticancer agent developed as a Bcl-2 inhibitor (Non Patent Document 5: Tse et al., 2008), and was also consistent with the finding of Adler et al. described above (Bcl-2 enhancement is a cause of anti-apoptosis capacity of senescent cells). ABT263 treatment also alleviated aging-relevant dysfunctions and artheriosclerosis in mice models (Non Patent Document 4: Chang et al., 2016, Non Patent Document 6: Childs et al., 2016).

There are reports on a senolytic drug other than the Bcl-2 inhibitor (ABT263) described above. Yi Zhu et al. (JL Kirkland's group) found that the combination of D + Q (dasatinib + quercetin) from 46 agents obtained by screening based on information such as senescent cell transcriptome analysis exhibits a senolytic effect. Dasatinib is an anticancer agent that acts as an inhibitor of tyrosine kinase, such as BCR-ABL, and quercetin is a type of flavonoid and a PI3K kinase inhibitor (Non Patent Document 7: Zhu et al., 2015).

In addition, two groups simultaneously found a senolytic effect of a cardiac glycoside (Non Patent Document 8: Guerrero et al., 2019, Non Patent Document 9: Triana-Martinez et al., 2019). The cardiac glycoside was originally known as a therapeutic drug for heart failure, which potentiates myocardial cells by inhibiting a cell surface Na+/K+ pump. In senescent cells, inhibition of the Na+/K+ pump was found to induce NOXA (a Bcl-2 homologue) and induce apoptosis.

On the other hand, glycolytic metabolism is a general term for the metabolic process of synthesizing a high-energy molecule known as ATP by degrading glucose taken up by cells. There are two pathways in glycolytic metabolism: a process that synthesizes lactate from the glycolytic intermediate pyruvate; and a process of proceeding to oxidative phosphorylation in mitochondria. The former is called anaerobic glycolytic metabolism because it does not require oxygen respiration, and the latter is called aerobic glycolytic metabolism because it synthesizes ATP by mitochondrial oxygen respiration. This glycolytic metabolism plays an important role in energy metabolism in most of the normal cells and is essential for the survival of individual cells. Because of this, an abnormality in glycolytic metabolism is closely related to human diseases.

A decrease in glycolytic metabolism causes neurodegenerative diseases, cardiac dysfunction, diabetes, muscle atrophy, anemia, or the like, whereas some pathological conditions in which glycolytic metabolism is enhanced are known. Examples of the latter conditions include cancer, ischemia (decreased blood flow in tissues), and local inflammation. In particular, in many cancer cells, glycolytic metabolism is enhanced (Warburg effect; Non Patent Document 10; Warburg O., Science, 1956, 124: p.269-270), which is known as one of the biological characteristics of cancer cells. In fact, in current clinical practice, FDG (fluorodeoxyglucose)-PET scan based on this property is useful for evaluating the size of and detecting the metastasis of tumors in the body of a patients.

The glycolytic metabolic pathway consists of sequential reactions of 10 glycolytic enzymes. The glycolytic enzyme phosphoglycerate mutase PGAM, as the eighth enzyme of the 10 glycolytic enzymes, catalyzes the conversion reaction of 3-phosphoglycerate (3PG) to 2-phosphoglycerate (2PG). The present inventors have found that overexpression of PGAM in cells induces glycolysis enhancement and an oxidative stress reduction effect to make the cells less susceptible to senescence, whereas deactivation of PGAM induces stress-induced senescence (Non Patent Document 11: Kondoh et al., 2005, Non Patent Document 12: Mikawa et al., 2014). In addition, the present inventors have found that PGAM binds to Chk1, which is a serine-threonine protein kinase, in cells, that this physical binding plays an important role in glycolytic metabolism, and that the glycolysis can be controlled by modulating this physical binding. Furthermore, the present inventors have also successfully found novel glycolytic metabolic control substances such as a substance that specifically inhibit glycolytic metabolism in cancers and a substance that exhibits an anti-aging effect in cells or a tissue in which cellular senescence is to be suppressed (Patent Document 1).

PRIOR ART DOCUMENTS

Patent Document

Patent Document 1: JP 2018-194299 A

Non Patent Document

Non Patent Document 1: Adler et al., Genes Dev., 2007, 21, 3244-3257
Non Patent Document 2: Coppe et al., PLoS Biol., 2008, 6, 2853-2868
Non Patent Document 3: Baker et al., Nature, 2011, 479, 232-236
Non Patent Document 4: Chang et al., Nat. Med., 2016, 22, 78-83
Non Patent Document 5: Tse et al., Cancer Res., 2008, 68, 3421-3428
Non Patent Document 6: Childs et al., Science, 2016, 354, 472-477
Non Patent Document 7: Zhu et al., Aging Cell, 2015, 14, 644-658
Non Patent Document 8: Guerrero et al., Nat Metab. 2019, 1, 1074-1088
Non Patent Document 9: Triana-Martinez et al., Nat. Commun., 2019, 10, 4731
Non Patent Document 10: Warburg O., Science, 1956, 124, 269-270
Non Patent Document 11: Kondoh et al., Cancer Res., 2005, 65, 177-185
Non Patent Document 12: Mikawa et al., J. Cell Biol., 2014, 204, 729-745

SUMMARY OF INVENTION

Technical Problem

Under the circumstances as described above, objects of the present invention are to provide a screening method that can efficiently find a novel senolytic drug, and to provide a senolytic drug obtained by the screening method.

Solution to Problem

The present inventors have carried out intensive research in order to solve the above problems, and as a result, as a screening method for efficiently finding a novel senolytic drug, established a screening method including a primary screening step of measuring inhibitory activity of a test substance on physical binding between PGAM and ChkI and a secondary screening step of measuring selective senolytic activity thereof. Then, by this screening method, the present inventors have found a large number of novel senolytic drugs. Furthermore, the present inventors have carried out analysis of a downstream transcription factor of the PGAM1-Chk1 pathway, and as a result, revealed that the transcription factor HIF-2$\alpha$ is a downstream target of PGAM1-Chk1 binding and found that an HIF-2$\alpha$ siRNA also has senolytic activity. This has revealed that regulation of HIF-2$\alpha$ controlling signals (including acetylation and ubiquitination), in addition to inhibition of PGAM1-Chk1 binding, can also induce senolysis via inactivation of HIF-2$\alpha$. Furthermore, it has also been revealed that the transcription factor HIF-2$\alpha$ acts in such a way as to suppress the expression of the apoptosis-inducing gene BIM. That is, the present invention is as shown below.

[1] A senolytic drug that inhibits binding between PGAM and Chk1 and selectively kills senescent cells.
[2] The senolytic drug according to [1], wherein the senolytic drug comprises, as an active ingredient, at least one selected from the group consisting of an RSK1 kinase inhibitor, an Fak kinase inhibitor, an inhibitor of a signal pathway involving Fak kinase, a CDK inhibitor, a calcium antagonist, a cardiac glycoside, a DNA-damaging agent, an antimicrobial agent, an Aurora kinase inhibitor, a flavonoid, a PI3K kinase inhibitor, an HDAC inhibitor, a therapeutic agent for age-related macular degeneration, a p38 MAPK inhibitor, an mTOR inhibitor, a tyrosine kinase inhibitor, a Bcl-2 inhibitor, a statin, a serotonin receptor antagonist, a further kinase inhibitor, and nutlin 3b.
[3] The senolytic drug according to [2], wherein

the RSK1 kinase inhibitor is an RSK1 antisense nucleic acid, an RSK1 siRNA, or BID1870;
the Fak kinase inhibitor is PF-00562271 or PF-431396;
the inhibitor of a signal pathway involving Fak kinase is PHA-665752 or CP-100356;
the CDK inhibitor is SNS-032, AZD5438, Flavopiridol (Alvocidib), PHA-793887, AT7519, or PHA-767491;
the calcium antagonist is Manidipine or Lomerizine 2HCl;
the cardiac glycoside is Proscillaridin A or Digoxin;
the DNA-damaging agent is Doxorubicin (Adriamycin) HCl, Daunorubicin HCl, Epirubicin HCl, Mitoxantrone 2HCl, or Hydroxy Camptothecine;
the antimicrobial agent is Aminoacridine, Triclosan, or Ethacridine lactate;
the Aurora kinase inhibitor is AMG-900 or JNJ-7706621;
the flavonoid is Chrysin;
the PI3K kinase inhibitor is PF-05212384 or XL147;
the HDAC inhibitor is Trichostatin A (TSA);
the therapeutic agent for age-related macular degeneration is Verteporfin;
the p38 MAPK inhibitor is SB203580 or Doramapimod (BIRB796);
the mTOR inhibitor is AZD8055 or KU-0063794;
the tyrosine kinase inhibitor is Neratinib, Vargatef, or NVP-BHG712;
the Bcl-2 inhibitor is TW-37;
the statin is Mevastatin;
the serotonin receptor antagonist is RS-127445; and
the further kinase inhibitor is BMS-345541 or CX-4945.

[4] A method for screening a senolytic drug, comprising:

(A) a step of measuring inhibitory activity of a test substance on binding between PGAM and Chk1; and
(B) a step of measuring selective senolytic activity of a test substance determined to have inhibitory activity on binding between PGAM and ChkI in step (A).

[5] The method according to [4], wherein step (B) comprises measuring toxic activity of a test substance on young cells and toxic activity thereof on senescent cells, and selecting a test substance having no toxic activity on young cells and having toxic activity on senescent cells.
[6] A senolytic drug that has an action of inactivating HIF-2$\alpha$ and selectively kills senescent cells.
[7] The senolytic drug according to [6], wherein the senolytic drug comprises an HIF-2$\alpha$ inhibitor as an active ingredient.

[8] The senolytic drug according to [7], wherein the HIF-2$\alpha$ inhibitor is an antisense nucleic acid or siRNA of HIF-2$\alpha$.

[9] A method for screening a senolytic drug, comprising:

(X) a step of measuring HIF-2$\alpha$ inhibitory activity of a test substance; and

(Y) a step of measuring selective senolytic activity of a test substance determined to have HIF-2$\alpha$ inhibitory activity in step (X).

[10] The method according to [9], wherein step (Y) comprises measuring toxic activity of a test substance on young cells and toxic activity thereof on senescent cells, and selecting a test substance having no toxic activity on young cells and having toxic activity on senescent cells.

Advantageous Effects of Invention

The method for screening a senolytic drug according to the present invention can remarkably improve the efficiency of the screening of a senolytic drug by carrying out primary screening including a step of measuring inhibitory activity on binding between PGAM and Chk1 before the step of measuring selective senolytic activity. The present inventors have found that the binding between PGAM and Chkl was not observed in young normal cells, but was remarkably enhanced in senescent cells, thereby causing the occurrence of enhanced glycolytic metabolism in senescent cells. Accordingly, by first screening many substances for a substance having inhibitory activity on binding between PGAM and Chk1, a substance that can inhibit the binding between PGAM and Chkl in senescent cells can be easily obtained, and by measuring the selective senolytic activity of such a substance, a senolytic drug can be efficiently found. In fact, the screening method of the present invention was used to narrow down a library of more than 2300 existing drugs having established safety to about 200 candidate drugs by primary screening, and a selective senolytic activity test was carried out to finally succeed in identifying about 40 novel senolytic drugs. Research by the present inventors has also confirmed that in stress-induced senescence, which is telomere-independent cellular senescence, glycolytic metabolism enhancement due to the binding between PGAM and Chkl occurs, as in so-called senescent cells, and according to the screening method of the present invention, it is also possible to find a substance that exhibits a therapeutic effect not only on an age-related disease but also on a disease developed by stress-induced senescence. Furthermore, the present inventors have found that the transcription factor HIF-2$\alpha$ is a downstream target of the PGAM1-Chk1 binding, and thus revealed that senolysis can be induced by controlling HIF-2$\alpha$. Based on this new finding, screening for an agent that inactivates HIF-2$\alpha$ has also made it possible to find a more effective senolytic drug.

BRIEF DESCRIPTION OF DRAWINGS

[Fig. 1] Fig. 1 is an explanatory diagram of an experimental system that can measure PGAM-Chkl binding in cells in which cellular senescence can be induced by a tamoxifen agent.

[Fig. 2] Fig. 2 is a diagram showing changes over time in amount of senescence marker proteins when cellular senescence is induced.

[Fig. 3] Fig. 3 is a diagram showing changes over time in senescence marker SA-$\beta$-Gal staining and PGAM-Chkl binding when cellular senescence is induced.

[Fig. 4] Fig. 4 is diagrams showing changes over time in glycolytic metabolism when cellular senescence is induced. The upper diagram shows changes in lactate production, and the lower diagram shows changes in sugar uptake rate.

[Fig. 5] Fig. 5 is diagrams showing the chemical structures of nutlin 3a and nutlin 3b.

[Fig. 6] Fig. 6 is a diagram showing the inhibitory effects of nutlin 3a and nutlin 3b on PGAM-Chkl binding in senescent cells.

[Fig. 7] Fig. 7 is a diagram showing a suppressive effect of nutlin 3b treatment against PGAM-Chkl binding potentiation in senescent cells.

[Fig. 8] Fig. 8 is diagrams showing suppressive effects of nutlin 3a and nutlin 3b on glycolytic metabolism (sugar uptake and lactate production) in senescent cells.

[Fig. 9] Fig. 9 is a diagram showing inhibitory effects of nutlin 3a and nutlin 3b on PGAM-Chkl binding in cancer cells.

[Fig. 10] Fig. 10 is a diagram showing a comparison of p53 activation effects of nutlin 3a and nutlin 3b.

[Fig. 11] Fig. 11 is a diagram showing a comparison of senolytic effects of nutlin 3a and nutlin 3b.

[Fig. 12] Fig. 12 is a diagram showing a comparison of senolytic effects of nutlin 3a and nutlin 3b.

[Fig. 13] Fig. 13 is a diagram showing apoptosis of senescent cells by nutlin 3b treatment.

[Fig. 14-1] Fig. 14-1 is diagrams showing effects of nutlin 3b administration to aged mice.

[Fig. 14-2] Fig. 14-2 is diagrams showing effects of nutlin 3b administration to aged mice.

[Fig. 15] Fig. 15 is a diagram showing a suppressive effect of an RSK kinase inhibitor on S280 phosphorylation of Chkl kinase.

[Fig. 16] Fig. 16 is diagrams showing a comparison of PGAM-Chkl binding in S280A and S280D mutant strains of Chkl kinase in young cells and senescent cells.

[Fig. 17] Fig. 17 is a diagram showing enhanced phosphorylation of RSK1-Thr573 and enhanced phosphorylation of Chk1-Ser280 in senescent cells.

[Fig. 18] Fig. 18 is a diagram showing an effect of RSK1 siRNA treatment on senescent cells.

[Fig. 19] Fig. 19 is a diagram showing a senolytic effect of the RSK kinase inhibitor BI D1870.

[Fig. 20] Fig. 20 is diagrams showing a senolytic effect of an RSK1 siRNA.

[Fig. 21] Fig. 21 is a diagram showing a comparison of expression of the transcription factor HIF-2$\alpha$ expression in young cells and senescent cells.

[Fig. 22] Fig. 22 is diagrams showing reduction in HIF-2$\alpha$ protein by treatment with nutlin 3b and the RSK kinase inhibitor BI-D 1870.

[Fig. 23] Fig. 23 is a diagram showing an effect of HIF-2$\alpha$ siRNA treatment on young cells and senescent cells.

[Fig. 24] Fig. 24 is a diagram showing an effect of HIF-2$\alpha$ siRNA treatment on young cells and senescent cells.

[Fig. 25] Fig. 25 is a diagram showing analysis results of BIM gene expression in senescent cells subjected to nutlin 3b treatment.

[Fig. 26] Fig. 26 is a diagram showing analysis results of BIM gene expression in senescent cells subjected to HIF-2$\alpha$ knockdown.

[Fig. 27] Fig. 27 is a diagram showing a suppressive effect of BIM gene knockdown on senolysis.

[Fig. 28] Fig. 28 is a diagram showing an effect of BIM gene knockdown on senolysis by HIF-2$\alpha$ knockdown.

[Fig. 29] Fig. 29 is a diagram schematically showing the screening method of the present invention.

[Fig. 30] Fig. 30 is a diagram showing results of the primary screening of the present invention.

[Fig. 31-1] Fig. 31-1 is a diagram showing results of the secondary screening of the present invention.

[Fig. 31-2] Fig. 31-2 is a diagram showing results of the secondary screening of the present invention.

[Fig. 32] Fig. 32 is a diagram showing senolytic candidate drugs according to the screening method of the present invention.

[Fig. 33] Fig. 33 is a diagram showing the schedule of a test of nutlin 3b administration to aged mice.

[Fig. 34] Fig. 34 is diagrams showing effects of nutlin 3b administration on muscle strength (wire hang test) and renal function markers (plasma BUN, plasma creatinine).

[Fig. 35] Fig. 35 is diagrams showing effects of nutlin 3b administration on hepatic function marker (plasma albumin level) and plasma lactate level.

[Fig. 36A] Fig. 36A is diagrams showing results of histochemical analysis of the influence of nutlin 3b administration on the liver.

[Fig. 36B] Fig. 36B is diagrams showing a suppressive effect of nutlin 3b administration on SASP factor (IL6, CXCL1, TNF$\alpha$, and CCL5) mRNA expression in the liver.

[Fig. 37] Fig. 37 is diagrams showing an improving effect of nutlin 3b administration on cellular senescence in adipose tissue (SA-$\beta$-Gal staining).

[Fig. 38] Fig. 38 is a diagram showing that the proteasome inhibitor MG132 inhibits a suppressive effect of nutlin 3b treatment of senescent cells on expression of HIF-2$\alpha$ protein.

[Fig. 39] Fig. 39 is a diagram showing that nutlin 3b treatment of senescent cells does not vary expression of an HIF-2$\alpha$ mRNA.

[Fig. 40] Fig. 40 is a diagram showing the consensus sequence for phosphorylation by Chkl located at the N-terminus of HIF-2$\alpha$.

[Fig. 41] Fig. 41 is a diagram showing the influence of the S12A mutation and the S19A mutation of HIF-2$\alpha$ on HIF-2$\alpha$ protein stability in senescent cells.

[Fig. 42] Fig. 42 is a diagram showing the influence of the S12A mutation and the S19A mutation of HIF-2$\alpha$ on HIF-2$\alpha$ ubiquitination modification in senescent cells.

[Fig. 43] Fig. 43 is a diagram showing Chkl-dependent phosphorylation of the Ser12 residue of HIF-2$\alpha$ in senescent cells.

[Fig. 44] Fig. 44 is diagrams showing that PGAM-Chkl interaction in human fibroblasts is potentiated by addition of culture medium from senescent cells.

[Fig. 45] Fig. 45 is diagrams showing that accumulation of HIF-2$\alpha$ in human fibroblasts, phosphorylation of Ser12 of HIF-2$\alpha$, and potentiation of PGAM-Chkl interaction occur in a concentration-dependent manner of lactate.

[Fig. 46] Fig. 46 is a diagram showing the influence of lactate on SASP expression and p21 expression in human fibroblasts.

[Fig. 47] Fig. 47 is a diagram showing enhanced expression of a lactate receptor (GPR81) in senescent cells.

[Fig. 48] Fig. 48 is a diagram showing PGAM-Chkl binding inhibition by lactate receptor (GPR81) knockdown.

[Fig. 49] Fig. 49 is a diagram showing enhanced phosphorylation of RSK and Chkl by lactate treatment.

[Fig. 50] Fig. 50 is a diagram showing that the apoptosis inhibitor QVD-Oph restores the viability of senescent cells under nutlin 3b treatment.

[Fig. 51] Fig. 51 is a diagram showing results of transcriptome analysis of nutlin 3b-treated senescent cells.

[Fig. 52] Fig. 52 is a diagram showing results of transcriptome analysis of nutlin 3b-treated senescent cells.

[Fig. 53] Fig. 53 is a diagram showing that previously reported ChIP datasets were searched for transcription factors closely related to the results of transcriptome analysis of nutlin 3b-treated senescent cells by using ChIPAtlas.

[Fig. 54] Fig. 54 is a diagram showing FOXM1 target genes that are among the top 150 genes out of the genes downregulated by nutlin 3b treatment in senescent cells.

[Fig. 55] Fig. 55 is a diagram showing the top 10 downregulated genes out of 41 apoptosis control-related genes affected by nutlin 3b treatment in senescent cells.

[Fig. 56] Fig. 56 is diagrams showing an effect of survivin knockdown in senescent cells.

[Fig. 57] Fig. 57 is a diagram showing mRNA expression in senescent cells affected by nutlin 3b treatment.

[Fig. 58] Fig. 58 is a diagram showing results of analysis of differential expression (GSEA analysis) of FOX family transcription factor target genes due to nutlin 3b administration.

[Fig. 59] Fig. 59 is a diagram showing results of analysis of differential expression (GSEA analysis) of FOX family transcription factor target genes due to nutlin 3b administration.

[Fig. 60] Fig. 60 is a diagram showing differential expression of FOX family transcription factors due to nutlin 3b treatment.

[Fig. 61] Fig. 61 is a diagram showing decreased expression of FOXM1 and survivin due to HIF-2$\alpha$ knockdown.

## DESCRIPTION OF EMBODIMENTS

Hereinafter, the method for screening a senolytic drug and the senolytic drug of the present invention will be described in detail.

<Method for screening a Senolytic drug >

The method for screening a senolytic drug of the present invention is a method for finding a substance that inhibits binding between PGAM and Chk1 and selectively kills senescent cells, and is specifically characterized by including: (A) a step of measuring inhibitory activity of a test substance on binding between PGAM and Chk1; and (B) a step of measuring selective senolytic activity of a test substance determined to have inhibitory activity on binding between PGAM and Chk1 in step (A). The present inventors have found that the binding between PGAM and Chkl was not observed in young normal cells, but was remarkably enhanced in senescent cells, thereby causing the occurrence of enhanced glycolytic metabolism. Accordingly, by first screening many substances for a substance having inhibitory activity on binding between PGAM and Chk1, a substance that can inhibit the binding between PGAM and Chkl in senescent cells can be easily obtained, and by measuring the selective senolytic activity thereof, a senolytic drug can be efficiently found.

Currently, there is no universal marker for cellular senescence, and a marker expressed in senescent cells differs depending on the stimulus that induces cellular senescence, the cell type, the timing, or the like, and thus it is considered that in order to identify senescent cells, a plurality of factors related to cellular senescence should be investigated. Therefore, in order to clarify the technical contents of the present invention, senescent cells in the present invention are defined as follows. That is, in the present invention, the senescent cells refer to cells that satisfy at least one of the following conditions: irreversible arrest of proliferation of primary cells; a change in cell morphology such as expansion of the cytoplasm and a cell nucleus or flattening of cells due to decrease in normal cell function; positive SA-bGAL staining (classical cellular senescence marker); elevated p16Ink4A (cellular senescence marker); and positive H2AX$\gamma$ staining (DNA damage marker). The senescent cells in the present invention are a concept including both cells that have undergone senescence due to aging (so-called replicative senescence) and stress-induced senescent cells (cells that have be exposed to various biological stresses (radiation, mutagen, oxidative stress, and the like)). Experimentally, cells that have undergone premature senescence by artificial exposure to DNA damage can be used as senescent cells. On the other hand, the young (normal) cells refer to cells other than the above senescent cells and refer to cells that have not undergone senescence due to aging, or stress-induced senescence.

In the present invention, senolysis refers to selective cell death of senescent cells, and an agent that selectively kills senescent cells is referred to as a senolytic drug.

In the present invention, PGAM is an enzyme, referred to as phosphoglycerate mutase, that is responsible for the eighth reaction among the 10 sequential reactions of glycolytic enzymes in the glycolytic metabolic pathway and that catalyzes the conversion reaction of 3-phosphoglycerate (3PG) to 2-phosphoglycerate (2PG). PGAM is responsible for a connection between cellular senescence and canceration. PGAM has two isoforms, PGAM1 and PGAM2. PGAM1 and PGAM2 have different tissue expression patterns, but have high homology in terms of amino acid sequence, and are considered to be equivalent in function and enzyme activity. The amino acid sequences of human PGAM1 and PGAM2 are shown as SEQ ID NOs: 1 and 2, respectively, and the cDNA sequences are shown as SEQ ID NOs: 3 and 4, respectively, in the sequence listing.

In the present invention, Chkl is a serine-threonine protein kinase, and is a checkpoint kinase that plays a role in activating a DNA damage checkpoint in the G2 phase of the cell cycle. Chkl was found by the present inventors to also play an important role in glycolytic metabolism. The amino acid sequence of human Chkl is shown as SEQ ID NO: 5, and the cDNA sequence is shown as SEQ ID NO: 6, in the sequence listing.

Glycolytic metabolism in the present invention is a general term for the metabolic process of synthesizing ATP by degrading glucose taken up by cells. There are two pathways in glycolytic metabolism: a pathway that degrades glucose to pyruvate as an intermediate thereof to synthesize lactate, and a pathway that leads to mitochondrial oxidative phosphorylation. The former is called anaerobic glycolytic metabolism because it does not require oxygen respiration, and the latter is called aerobic glycolytic metabolism because it synthesizes ATP by mitochondrial oxygen respiration. This glycolytic metabolism plays an important role in energy metabolism in most of the normal cells and is essential for the survival of an individual.

Each step of the screening method of the present invention will be described. The screening method of the present invention includes: a step of measuring inhibitory activity of a test substance on binding between PGAM and Chk1 as step (A); and a step of measuring selective senolytic activity of a test substance determined to have inhibitory activity on binding between PGAM and Chkl in step (A) as step (B).

<Step (A)>

The present step is a step of measuring inhibitory activity of a test substance on binding between PGAM and Chkl. In the present step, any method may be adopted as long as the method can measure the inhibitory activity of a test substance on the binding between PGAM and Chk1, and examples thereof include a method involving measuring the binding level between PGAM and Chk1 in the presence and absence of a test substance, and calculating the inhibitory activity of the test substance on the binding between PGAM and Chk1 from the ratio of both binding levels. This method preferably includes the following steps 1) to 3).

1) A step of measuring a binding level between PGAM and Chkl when a test substance is added to a sample including PGAM and Chkl
2) A step of measuring a binding level between PGAM and Chkl when the test substance is not added
3) A step of calculating inhibitory activity on binding between PGAM and Chk1 by the following expression (i):

$$\{\text{measured value in step 2)} - \text{measured value in step 1)}\}/\text{measured value in 2)} \times 100 \ (\%) \ ... \ (i)$$

In step 1) of the screening method of the present invention, a test substance is first added to a sample including PGAM and Chk1 to contact the test substance with PGAM and/or Chk1.

PGAM and Chkl used in the screening method of the present invention are as described above. PGAM and Chkl used in the screening method of the present invention also include a protein functionally equivalent to the known PGAM and Chkl described above. Examples of such a protein include, but are not limited to, mutants, alleles, variants, and homologues of PGAM and Chk1, partial peptides of PGAM and Chk1, a fusion protein with a further protein, and one labeled with a tag.

Examples of the mutants of PGAM and Chkl in the present invention include a naturally occurring protein that consists of an amino acid sequence derived from the amino acid sequence described above by substituting, deleting, inserting, and/or adding one or more amino acids and that is functionally equivalent to a protein consisting of the amino acid sequence described above. In addition, examples of the mutant of each of PGAM and Chkl also include a protein that is encoded by a naturally occurring DNA that hybridizes with a DNA consisting of the base sequence described above under a stringent condition and that is functionally equivalent to the protein consisting of the amino acid sequence described above.

In the present invention, the number of amino acids to be mutated is not particularly limited, and is usually considered to be 30 amino acids or less, preferably 15 amino acids or less, more preferably 5 amino acids or less (for example, 3 amino acids or less). The amino acid residue to be mutated is desirably mutated to another amino acid that conserves the properties of an amino acid side chain. Examples of the properties of an amino acid side chain include a hydrophobic amino acid (A, I, L, M, F, P, W, Y, or V), a hydrophilic amino acid (R, D, N, C, E, Q, G, H, K, S, or T), an amino acid having an aliphatic side chain (G, A, V, L, I, or P), an amino acid having a hydroxyl group-containing side chain (S, T, or Y), an amino acid having a sulfur atom-containing side chain (C or M), an amino acid having a carboxylic acid- and amide-containing side chain (D, N, E, or Q), an amino acid having a base-containing side chain (R, K, or H), and an amino acid having an aromatic-containing side chain (H, F, Y, W) (each letter in parentheses represents a one-letter code of an amino acid). It is already known that a polypeptide having an amino acid sequence derived from a certain amino acid sequence by modification by deletion, addition, and/or substitution of one or more amino acid residues with another amino acid sequence maintains the biological activity thereof.

"Functionally equivalent" in the present invention means that the target protein has a biological function or a biochemical function equivalent to that of PGAM or Chk1. The biological function or biochemical function of PGAM is a function as a glycolytic enzyme, and specific examples thereof include the function of catalyzing the conversion reaction of 3-phosphoglycerate (3PG) to 2-phosphoglycerate (2PG). In addition, examples of the biological function or biochemical function of Chk1 include a function as a serine-threonine protein kinase.

Examples of a method well known to those skilled in the art for preparing a DNA encoding a protein functionally equivalent to the target protein include a method that uses a hybridization technique or a polymerase chain reaction (PCR) technique. That is, it can be common practice for those skilled in the art to isolate a DNA having high homology to PGAM or Chkl by using the base sequence of PGAM or Chkl or a portion thereof as a probe and using an oligonucleotide that specifically hybridizes to PGAM or Chkl as a primer. As described above, a DNA encoding a protein having a function equivalent to that of PGAM or Chk1, which can be isolated by a hybridization technique or a PCR technique, is also included in the DNA of the present invention.

The biological species from which PGAM and Chkl used in the screening method of the present invention are derived is not limited to a specific biological species. Examples thereof include a human, a monkey, a mouse, a rat, a guinea pig, a pig, a cow, a yeast, and an insect.

The state of PGAM and Chkl used in the screening method of the present invention is not particularly limited, and may be, for example, a purified state, an intracellularly expressed state, or a state expressed in a cell extract.

In the screening method of the present invention, tag-labeled PGAM and Chkl may be used, the tag-labeled PGAM and Chk1 are preferably antibodies thereagainst or substances that have resins that can specifically bind thereto, and examples thereof include a FLAG tag, an HA tag, a Myc tag, a His tag, and a GST tag. In addition, PGAM and Chk1 may be labeled with tags that can bind to each other, as described later.

The test substance in the present invention is not particularly limited, and examples thereof include a single substance such as a natural compound, an organic compound, an inorganic compound, a nucleic acid, a protein (including an antibody), or a peptide; an expression product of a compound library, a nucleic acid library, a peptide library, or a gene library; and an extract such as a cell extract, cell culture supernatant, a fermented microbial product, a marine organism extract, a plant extract, a prokaryotic cell extract, an eukaryotic single cell extract, or an animal cell extract. In addition, the test substance in the present invention may be a mixture of the above. In addition, these test substances can be labeled as necessary and used. Examples of the label include a radiolabel and a fluorescent label.

In addition, in the present invention, contacting is carried out according to the state of PGAM and Chk1. For example, if PGAM and Chkl are in a purified state, contacting can be carried out by adding a test substance to the purified preparation. In addition, if PGAM and Chkl are in an intracellularly expressed state or in a state expressed in a cell extract, contacting can be carried out by adding a test substance to a culture medium of cells or an extract of the cells, or by direct administration thereof to an experimental animal. When the test substance is a protein, contacting can also be carried out, for example, by introducing a vector including a DNA encoding the protein into cells expressing PGAM and Chk1, or adding the vector to an extract of cells expressing PGAM and Chkl. In addition, a two-hybrid method that uses, for example, a yeast or animal cells can also be used.

In the present invention, by adding a test substance to a sample including PGAM and Chk1, PGAM and/or Chkl may be contacted with the test substance. In addition, by adding a test substance to a sample including either PGAM or Chkl and then adding the other that has not been included, each of these may be contacted with the test substance.

In the present invention, next, the binding level between PGAM and Chkl is measured. In step 2) of the screening method of the present invention, the binding level between PGAM and Chkl when the test substance is not added to the sample including PGAM and Chkl is measured. Specifically, the binding levels between PGAM and Chkl when a test substance is added (when contacted) and when the test substance is not added (when not contacted) are measured, and the values thereof are compared in step 3) of the screening method of the present invention. If the binding level between PGAM and Chkl decreases when a test substance is added as compared with when the test substance is not added, it can be determined that the test substance has the effect of inhibiting the binding between PGAM and Chkl (has inhibitory activity on the binding between PGAM and Chkl). Then, it can be determined that such a test substance has the effect of suppressing/inhibiting glycolytic metabolism and has toxic activity on senescent cells.

Specific examples of a method for measuring the binding level between PGAM and Chk1 include the following method. PGAM2-FLAG protein is immunoprecipitated from a protein extract of cells co-expressing PGAM2-FLAG and Chk1-myc-His expression vectors, by a FLAG tag antibody conjugated to protein G agarose. At this time, Chk1-myc-His protein coprecipitated by binding to the PGAM2-FLAG protein is detected by Western blotting and quantified. Alternatively, the Chk1-myc-His protein is precipitated from the same protein extract, by a Ni-NTA agarose bead. At this time, PGAM2-FLAG protein coprecipitated by binding to the Chk1-myc-His protein is detected by Western blotting and quantified.

Examples of a preferred method for measuring the binding level between PGAM and Chk1 include, in addition to the above, a method that uses PGAM and Chk1 labeled with tags that can bind to each other, and specific examples thereof include the following method that uses a Nanobit system. In Nanobit, a large subunit and a small subunit of a photoprotein prepared based on a luciferase are attached as tags to PGAM and Chk1, respectively. These tags have structures that can bind to each other. Each subunit itself does not emit light, but the binding between PGAM and Chk1 causes the large subunit and the small subunit to associate to form a complete photoprotein, thereby emitting a luminescence signal. By measuring the intensity of the luminescence signal at this time, the binding level can be measured with high sensitivity and high quantitativity.

<Step (B)>

In the present step, the selective senolytic activity of a test substance determined to have inhibitory activity on binding between PGAM and Chk1 in step (A) above is measured, and a test substance having high selective senolytic activity is selected. Examples of a method for selecting a test substance having high selective senolytic activity include a method involving measuring toxic activity of a test substance on young cells and toxic activity thereof on senescent cells and selecting a test substance having no toxic activity on young cells and having toxic activity on senescent cells. In addition, the toxic activity of a test substance on young cells and the toxic activity thereof on senescent cells may be measured to select a test substance having remarkably higher toxic activity on senescent cells than the toxic activity on young cells.

Here, in the present invention, examples of the young cells as cultured mouse cells include primary mouse fibroblasts (MEFs; mouse embryonic fibroblasts, or REFs; rat embryonic fibroblasts) and primary mouse skin keratinocytes. In addition, in the present invention, examples of the young cells as cultured human cells include human embryonic lung fibroblasts (IMR90, WI-38, TIG, or MRC cells), primary human skin cells (NHDF cells, BJ cells, or NHEK cells), normal human mammary epithelial cells (NHMEC cells), normal human vascular endothelial cells (HUVEC cells, HAEC cells, HPAEC cells, or HCAEC cells), and normal human prostate epithelial cells (HPEC). Depending on the cell type, the speed of proliferation thereof differs, and the speed of senescence thereof also differs, but in general, it is known that mouse cells become senescent cells in about one month of continuous culture after initial establishment and that human cells become senescent cells in about three months thereof. Therefore, mouse cells within 2 to 3 weeks of continuous culture after initial establishment can be referred to as young cells, and human cells within 1 month of continuous culture after initial establishment can be referred to as young cells. On the other hand, in the present invention, examples of the senescent cells include those cultured beyond the continuous culture period described above, those cultured for 3 to 4 weeks or more for a mouse, and 1 to 2 months or more for a human. Furthermore, even young cells of a mouse or a human can also be caused to transition into senescent cells in a short period of time by a stress that induces cellular senescence (oxidative stress, carcinogenic stress, DNA damage stress, or the like).

<Senolytic drug>

As described above, according to the screening method of the present invention, a senolytic drug can be efficiently found, and the present inventors have actually succeeded in finding 40 or more novel senolytic drugs (compounds having senolytic activity). In addition, by analyzing the mechanism of PGAM-Chkl binding, the present inventors have found that S280 phosphorylation of Chkl kinase by RSK1 kinase, which is a downstream signal kinase of oncogenic Ras mutation, is important for PGAM-Chkl binding. Based on this novel finding, it was able to be confirmed that an RSK1 kinase inhibitor inhibits PGAM-Chkl binding and also has selective toxic activity on senescent cells, and it was revealed that an RSK1 kinase inhibitor exhibits an excellent senolytic effect.

That is, the senolytic drug of the present invention is a medicament that inhibits the binding between PGAM and Chkl and selectively kills senescent cells, and contains such a compound as an active ingredient. Specifically, the senolytic drug of the present invention contains, as an active ingredient, at least one selected from the group consisting of an RSK1 kinase inhibitor, an Fak kinase inhibitor, an inhibitor of a signal pathway involving Fak kinase, a CDK inhibitor, a calcium antagonist, a cardiac glycoside, a DNA-damaging agent, an antimicrobial agent, an Aurora kinase inhibitor, a flavonoid, a PI3K kinase inhibitor, an HDAC inhibitor, a therapeutic agent for age-related macular degeneration, a p38 MAPK inhibitor, an mTOR inhibitor, a tyrosine kinase inhibitor, a Bcl-2 inhibitor, a statin, a serotonin receptor antagonist, a further kinase inhibitor, and nutlin 3b.

(RSK1 kinase inhibitor)

RSK1 is an RSK (ribosomal S6 kinase) family member and a growth factor-regulated serine-threonine kinase. RSK1 has two different kinase catalytic domains and phosphorylates a variety of substrates, including a member of the mitogen-activated kinase (MAPK) signaling pathway. The RSK1 kinase inhibitor contained in the senolytic drug of the present invention is not particularly limited as long as the inhibitor is a substance that inhibits the above RSK1 kinase activity, and preferred examples thereof include a compound such as an antisense nucleic acid complementary to a transcript of a DNA encoding RSK1, an siRNA, or BI-D1870 (CAS number: 501437-28-1).

The antisense nucleic acid used in the present invention is preferably an antisense nucleic acid that suppresses the expression of the RSK1 gene by inhibiting the process of transcription, splicing, or translation. As an embodiment of an antisense sequence, designing an antisense sequence complementary to an untranslated region in the vicinity of the 5' end of an RSK1 mRNA is considered to be effective in inhibiting translation of the gene. However, a sequence complementary to a coding region or a 3'-untranslated region can also be used. As described above, sequences that can be used in the present invention include not only sequences complementary to a translated region but also those complementary to an untranslated region of the gene can be used. As described above, nucleic acids used in the present invention include not only a nucleic acid including an antisense sequence of a translated region but also a nucleic acid including an untranslated region of the gene. The antisense nucleic acid has preferably 90% or more, most preferably 95% or more complementarity to a transcript of the target gene. The length of an antisense RNA that effectively inhibits the expression of the target gene by using an antisense sequence is not particularly limited.

The RSK1 siRNA used in the present invention means a double-stranded RNA consisting of a short chain within a range that does not exhibit toxicity in cells, and can be, for example, 15 to 49 base pairs, preferably 15 to 35 base pairs, and more preferably 21 to 30 base pairs in length.

The RSK1 siRNA need not be completely identical to the RSK1 gene, and has at least 70% or more, preferably 80% or more, more preferably 90% or more, and most preferably 95% or more sequence homology. A double-stranded RNA portion in which RNAs are paired in the siRNA is not limited to one in which RNAs are perfectly paired, and may include an unpaired portion due to a mismatch (a corresponding base is not complementary), a bulge (one strand has no corresponding base), or the like. In the present invention, both a bulge and a mismatch may be included in a double-stranded RNA region in which RNAs are paired in a dsRNA.

In the present invention, examples of the RSK1 siRNA include one including the sequence of SEQ ID NO: 7 or SEQ ID NO: 8. The siRNA in the present invention may have two overhanging bases such as dTdT added to the 3' end for the purpose of improving the resistance to nuclease degradation or the like.

(Fak kinase inhibitor)

Fak (focal adhesion kinase) is a non-receptor protein tyrosine kinase and is involved in signaling from an integrin-enriched focal adhesion that mediates cell adhesion with the extracellular matrix. An FAK-promoted signal is known to be involved in the survival of anchorage-dependent cells and to be very important for efficient cell migration in response to growth factor receptor or integrin stimulation. The Fak kinase inhibitor contained in the senolytic drug of the present invention is not particularly limited as long as the inhibitor is a substance that inhibits the above Fak kinase activity, and preferred examples thereof include PF-00562271 (CAS No. 939791-41-0), PF-431396 (CAS No. 717906-29-1), and salts or derivatives thereof having activity equivalent to that thereof.

(Inhibitor of signal pathway involving Fak kinase)

Examples of the inhibitor of the signal pathway involving Fak kinase contained in the senolytic drug of the present invention include a c-Met inhibitor and an Mdr-1 inhibitor. Preferred examples of the c-Met inhibitor contained in the senolytic drug of the present invention include PHA-665752 (CAS No. 477575-56-7), and a salt or a derivative thereof having activity equivalent to that of the compound. In addition, preferred examples of the Mdr-1 inhibitor contained in the senolytic drug of the present invention include CP-100356 (CAS No. 142715-48-8), and a salt or a derivative thereof that is a compound having activity equivalent to that thereof.

(CDK inhibitor)

Cyclin-dependent kinase (CDK) is a protein that is active only when the protein binds to a cyclin in a group of intranuclear kinases that govern the cell cycle. There are a plurality of CDKs that are activated at different time points in the cell cycle. The activity is controlled by phosphorylation and dephosphorylation of tyrosine and threonine residues of the CDK itself. The CDK inhibitor contained in the senolytic drug of the present invention is a substance that inhibits or suppresses the activity of such a CDK, and preferred examples thereof include SNS-032 (CDK2/7/9 inhibitor; CAS No. 345627-80-7), AZD5438 (CDK1/2/9 inhibitor; CAS No. 602306-29-6), Flavopiridol (Alvocidib) (CDK1/2/4/6/9 inhibitor; CAS No. 146426-40-6), PHA-793887 (CDK7/9 inhibitor; CAS No. 718630-59-2), AT7519 (CDK1/2/4/6/9 Inhibitor; CAS No. 844442-38-2), PHA-767491 (CDK7/9 inhibitor; CAS No. 942425-68-5), and salts or derivatives thereof that are compounds having activity equivalent to that thereof.

(Calcium antagonist)

The calcium antagonist is an agent that binds to a calcium channel on a cell membrane and inhibits a calcium ion influx into cells. Preferred examples of the calcium antagonist contained in the senolytic drug of the present invention include Manidipine (CAS No. 89226-50-6), Lomerizine 2HCl (CAS No. 101477-54-7), and salts or derivatives thereof that are compounds having activity equivalent to that thereof.

(Cardiac glycoside)

The cardiac glycoside is a general term for a steroid glycoside used for supraventricular tachycardia such as atrial fibrillation or atrial flutter, congestive heart failure accompanied by edema, or arrhythmia, and preferred examples of the cardiac glycoside contained in the senolytic drug of the present invention include Proscillaridin A (CAS No. 466-06-8), Digoxin (CAS No. 20830-75-5), and salts or derivatives thereof that are compounds having activity equivalent to that thereof.

(DNA-damaging anticancer agent)

ADNA-damaging anticancer drug is a general term for a type of anti-cancer drug that generates DNA- damagein cancer cells to selectively lead to cell death, and preferred examples of the DNA-damaging anticancer drug contained in the senolytic drug of the present invention include Doxorubicin (Adriamycin) HCl (CAS No. 25316-40-9), Daunorubicin HCl (CAS No. 23541-50-6), Epirubicin HCl (CAS No. 56390-09-1), Mitoxantrone 2HCl (CAS No. 70476-82-3), Hydroxy Camptothecine (CAS No. 64439-81-2), salts or derivatives thereof that are compounds having activity equivalent to that thereof.

(Antimicrobial agent)

Preferred examples of the antimicrobial agent contained in the senolytic drug of the present invention include Aminoacridine (CAS No. 90-45-9), Triclosan (CAS No. 3380-34-5), Ethacridine lactate (TAZ activation; CAS No. 1837-57-6), and salts or derivatives thereof that are compounds having activity equivalent to that thereof.

(Aurora kinase inhibitor)

Aurora kinase is an enzyme encoded by the AURKA gene in humans and is a member of the serine/threonine kinase family. Aurora kinase contributes to precise progression of mitosis and meiosis and is essential for cell proliferation.. It is known that Aurora kinase activity which is regulated by phosphorylation at one or more site peaked at G2/M phase transition of the cell cycle. Preferred examples of the Aurora kinase inhibitor contained in the senolytic drug of the present invention include AMG-900 (CAS No. 945595-80-2), JNJ-7706621 (CDK1, 2, Aurora A, B inhibition; CAS No. 443797-96-4), and salts or derivatives thereof that are compounds having activity equivalent to that thereof.

(Nutlin-3b)

Nutlin-3b (CAS No. 675576-97-3) is an optical isomer of Nutlin-3a. The senolytic drug of the present invention preferably contains Nutlin-3b (CAS No. 675576-97-3).
In addition to the above, a flavonoid (Chrysin; CAS No. 480-40-0), a PI3K kinase inhibitor (PF-05212384; CAS No. 1197160-78-3, XL147; CAS No. 934526-89-3), an HDAC inhibitor (Trichostatin A (TSA); CAS No. 58880-19-6), a therapeutic agent for age-related macular degeneration (Verteporfin; CAS No. 129497-78-5), a p38 MAPK inhibitor (SB203580; CAS No. 152121-47-6, Doramapimod (BIRB 796); CAS No. 285983-48-4), an mTOR inhibitor (AZD8055; CAS No. 1009298-09-2, KU-0063794; CAS No. 938440-64-3), a tyrosine kinase inhibitor (Neratinib (HER2, EGFR inhibition); CAS No. 698387-09-6, Vargatef, CAS No. 656247-17-5, NVP-BHG712 (c-Raf, c-Src inhibition); CAS No. 940310-85-0), a Bcl-2 inhibitor (TW-37; CAS No. 877877-35-5), a statin (Mevastatin; CAS No. 73573-88-3), a serotonin receptor antagonist (RS-127445; CAS No. 199864-87-4), and a further kinase inhibitor (BMS-345541; CAS No. 445430-58-0, CX-4945; CAS No. 1009820-21-6) have senolytic activity, and thus these compounds, and salts or derivatives thereof that are compounds having senolytic activity equivalent to that thereof can be adopted as active ingredients of the senolytic drug of the present invention.

In addition to the above active ingredients, the senolytic drug of the present invention may contain, as other optional ingredients, one or more pharmaceutically, pharmacologically or physiologically acceptable carriers, excipients, binders, disintegrants, lubricants, dispersants, surfactants, plasticizers, suspending agents, emulsifiers, diluents, buffering agents, antioxidants, bacterial inhibitors, or the like. In addition, the senolytic drug may further include a low-molecular-weight polypeptide, a protein such as serum albumin, gelatin, or an immunoglobulin, an amino acid, a sugar such as a polysaccharide and a monosaccharide, a carbohydrate, or a sugar alcohol. The senolytic drug of the present invention may further contain other active ingredients as necessary.

The senolytic drug of the present invention can be produced by any conventionally known method in the field of a pharmaceutical, a quasi-drug, a food, or the like. In the production process thereof, the above active ingredients and other optional ingredients may be added and mixed by any known method.

When formed into an aqueous solution for injection is obtained, the senolytic drug may be used in combination with, for example, an isotonic solution including physiological saline, glucose or other adjuvants such as D-sorbitol, D-mannose, D-mannitol, or sodium chloride, or a suitable dissolution aid such as an alcohol (ethanol or the like), a polyalcohol (propylene glycol, PEG, or the like), or a nonionic surfactant (polysorbate 80, HCO-50). A diluent, a dissolution aid, a pH adjuster, a soothing agent, a sulfur-containing reducing agent, an oxidation inhibitor, or the like may be further contained as necessary.

In addition, the senolytic drug can also be encapsulated in a microcapsule (microcapsule of hydroxymethylcellulose, gelatin, poly[methylmethacrylic acid], or the like), or formed into a colloidal drug delivery system (liposome, albumin microsphere, microemulsion, nanoparticle, nanocapsule, or the like). Furthermore, a method for forming an agent into a sustained-release agent is also known and can be applied to the present invention.

When the senolytic drug of the present invention is used as a medicament for humans or other animals, other than direct administration of them to a patient, it is also possible to formulate the senolytic drug as a formulation by a known pharmaceutical method and administer the formulation. In the case of the formulation thereof, the pharmaceutically acceptable ingredients described above may be added.

Every agent in the present invention can be administered in the form of a pharmaceutical, and can be administered orally or parenterally systemically or locally. For example, intravenous injection such as drip infusion, intramuscular injection, intraperitoneal injection, subcutaneous injection, a suppository, enema, or an oral enteric preparation can be selected, and the administration method can be appropriately selected according to the age and the symptom of the patient. The effective dosage is selected in the range of 0.001 mg to 100 mg per kg body weight per dose. Alternatively, a dosage of 0.1 to 1000 mg, preferably 0.1 to 50 mg per patient can be selected. As a specific example, 0.1 mg to 40 mg, preferably 1 mg to 20 mg per kg of body weight per month (4 weeks) is divided into one to several times, and administered by a method such as intravenous injection such as drip infusion, subcutaneous injection, or oral administration, on an administration schedule of, for example, twice/week, once/week, once/2 weeks, or once/4 weeks. The administration schedule can also be adjusted by, for example, extending the administration interval from twice/week, or once/week to once/2 weeks, once/3 weeks, once/4 weeks, or the like while monitoring the physical conditions and monitoring trends in blood test values after administration.

<Senolytic drug 2>

Furthermore, as a result of the further analysis of a molecular mechanism of the PGAM1-Chk1 pathway, it is revealed that the transcription factor HIF-2$\alpha$ is regulated by PGAM1-Chk1 pathway and found that the HIF-2$\alpha$ siRNA also show senolytic activity. This has revealed that modulation of HIF-2$\alpha$ activity via regulatory signals (including acetylation and ubiquitination), in addition to inhibition of PGAM1-Chk1 binding, can also induce senolysis via inactivation of HIF-2$\alpha$. Furthermore, it has also been revealed that the transcription factor HIF-2$\alpha$ acts in such a way as to suppress the expression of the apoptosis-inducing gene BIM. Based on these results, the present invention also includes a senolytic drug that has the action of inactivating HIF-2$\alpha$ and selectively eliminates senescent cells. Specifically, the senolytic drug is a senolytic drug containing an HIF-2$\alpha$ inhibitor as an active ingredient.

HIF-2$\alpha$ is a functional homologue of the transcription factor HIF-1$\alpha$ and is evolutionarily conserved. An HIF-family protein is activated when cells or tissues are exposed to a hypoxic condition. In a hypoxic condition, mitochondrial oxygen respiration is limited, thus forcing cells to rely on glycolytic metabolism for energy production. In a hypoxic environment, activated HIF protein enhances a glycolytic metabolism via upregulation of glycolytic enzymes (Iyer et al., Genes and Development, 1998, Vol 12, p149-62). In a normal oxygen environment, HIF protein is an unstable protein that is susceptible to ubiquitination and degraded, whereas in a hypoxic condition, HIF ubiquitination is inhibited, which stabilizes the protein and enhances the activity. The HIF-2$\alpha$ inhibitor contained in the senolytic drug of the present invention is not particularly limited as long as the inhibitor is a substance that inhibits the above HIF-2$\alpha$ function, and preferred examples thereof include an antisense nucleic acid complementary to a transcript of a DNA encoding HIF-2$\alpha$, and an siRNA (for example, SEQ ID NO: 9 or SEQ ID NO: 10). For the antisense nucleic acids and siRNAs used in the present invention, in the description in the section on RSK1 kinase inhibitor above, RSK1 can be read as HIF-2α and applied as it is.

The description of the contents common to general medicaments in <Senolytic drug> above can also be applied to <Senolytic drug 2>.

<Method for screening a senolytic drug 2>

Furthermore, the present invention also includes a method for screening a senolytic drug, including: (X) a step of measuring HIF-2α inhibitory activity of a test substance; and (Y) a step of measuring selective senolytic activity of a test substance determined to have HIF-2α inhibitory activity in step (X). Step (Y) is characterized by measuring toxic activity of a test substance on young cells and toxic activity thereof on senescent cells, and selecting a test substance having no toxic activity on young cells and having toxic activity on senescent cells, and to a specific description thereof, the description of step (B) in <Method for screening a senolytic drug > can be applied as it is. In step (X), the HIF-2α inhibitory activity includes not only the activity of inhibiting the action of HIF-2α but also the activity of suppressing the expression of HIF-2α.

<Senolytic drug 3>

As described above, nutlin 3b has excellent senolytic activity. The present inventors have further investigated the effect of nutlin 3b, and found that the expression of CCNB1, CCNB2, PLK1, ASPM, CEP55, and BIRC5 (Survivin) is upregulated in senescent cells and suppressed by nutlin 3b treatment. In particular, BIRC5 (Survivin) was remarkably upregulated. From this result, it can be said that inhibitors against CCNB1, CCNB2, PLK1, ASPM, CEP55, and BIRC5 (Survivin), especially inhibitors against BIRC5 (Survivin) thereamong, have excellent senolysis activity. Examples of the inhibitors of CCNB1, CCNB2, PLK1, ASPM, CEP55, and BIRC5 include antisense nucleic acids complementary to transcripts of DNAs encoding these and siRNAs. Based on these results, the present invention also includes a senolytic drug that has the action of inactivating CCNB1, CCNB2, PLK1, ASPM, CEP55, and/or BIRC5 and selectively eliminates senescent cells. Specifically, the senolytic drug is a senolytic drug containing an inhibitor of CCNB1, CCNB2, PLK1, ASPM, CEP55, and/or BIRC5 as an active ingredient.

The description of the contents common to general medicaments in <Senolytic drug> above can also be applied to <Senolytic drug 3>.

<Method for screening a senolytic drug 3>

Furthermore, the present invention also includes a method for screening a senolytic drug including: (X) a step of measuring inhibitory activity of a test substance on CCNB1, CCNB2, PLK1, ASPM, CEP55, and/or BIRC5; and (Y) a step of measuring selective senolytic activity of a test substance determined to have inhibitory activity on CCNB1, CCNB2, PLK1, ASPM, CEP55, and/or BIRC5 in step (X). Step (Y) is characterized by measuring toxic activity of a test substance on young cells and toxic activity thereof on senescent cells, and selecting a test substance having no toxic activity on young cells and having toxic activity on senescent cells, and to a specific description thereof, the description of step (B) in <Method for screening a senolytic drug > can be applied as it is. In step (X) above, the inhibitory activity on CCNB1, CCNB2, PLK1, ASPM, CEP55, and/or BIRC5 includes not only the activity of inhibiting the action of CCNB1, CCNB2, PLK1, ASPM, CEP55, and/or BIRC5 but also the activity of suppressing the expression of CCNB1, CCNB2, PLK1, ASPM, CEP55, and/or BIRC5.

<Senolytic drug 4>

Further research on nutlin 3b has revealed that the expression of a specific FOX family gene is deeply involved in senolysis by nutlin 3b. The expression of FOXM1, FOXF1, FOXC1, FOXL1, FOXL2, FOXD1, FOXB2, FOXI2, and FOXN1 was downregulated by nutlin 3b treatment. In particular, the expression of FOXM1 was remarkably downregulated. In addition, the expression of FOXO1, FOXP1, FOXH1, FOXF2, FOXO4, FOXO3, FOXJ3, FOXQ1, FOXK2, FOXN3, FOXO6, FOXK1, FOXP4, FOXN2, and FOXJ2 was upregulated by nutlin 3b treatment. In particular, the expression of FOXO1 and FOXP1 was remarkably upregulated. From these results, it can be said that substances that control the expression of the above specific FOX family genes, especially thereamong a substance that controls the expression of the FOXM1, the FOXO family including FOXO1 and the FOXP family including FOXP1, particularly a substance that downregulates the expression of FOXM1, and a substance that upregulates the expression of the FOXO family including FOXO1 and the FOXP family including FOXP1 have excellent senolytic activity.

For example, examples of the substance that downregulates the expression of FOXM1 or the like include an antisense nucleic acid complementary to a transcript of a DNA encoding FOXM1 or the like, and an siRNA. That is, the present invention also includes a senolytic drug that has the action of inactivating FOXM1, FOXF1, FOXC1, FOXL1, FOXL2, FOXD1, FOXB2, FOXI2, and/or FOXN1, especially, FOXM1, and selectively eliminates senescent cells. Specifically, this senolytic drug is a senolytic drug containing inhibitors of FOXM1, FOXF1, FOXC1, FOXL1, FOXL2, FOXD1, FOXB2, FOXI2, and/or FOXN1, especially an inhibitor of FOXM1 as an active ingredient.

Furthermore, the present invention also includes a senolytic drug that has the action of activating FOXO1, FOXP1, FOXH1, FOXF2, FOXO4, FOXO3, FOXJ3, FOXQ1, FOXK2, FOXN3, FOXO6, FOXK1, FOXP4, FOXN2, and/or FOXJ2, especially thereamong, FOXO family including FOXO1, and/or the FOXP family including FOXP1, and selectively eliminates senescent cells. Specifically, this senolytic drug is a senolytic drug containing an activator of FOXO1, FOXP1, FOXH1, FOXF2, FOXO4, FOXO3, FOXJ3, FOXQ1, FOXK2, FOXN3, FOXO6, FOXK1, FOXP4, FOXN2, and/or FOXJ2, especially thereamong, FOXO family including FOXO1, and/or the FOXP family including FOXP1, as an active ingredient.

The description of the contents common to general medicaments in <Senolytic drug> above can also be applied to <Senolytic drug 4>.

<Method for screening a senolytic drug 4>

Furthermore, the present invention also includes a method for screening a senolytic drug including: (X) a step of measuring inhibitory activity of a test substance on FOXM1, FOXF1, FOXC1, FOXL1, FOXL2, FOXD1, FOXB2, FOXI2, and/or FOXN1; and (Y) a step of measuring selective senolytic activity of a test substance determined to have inhibitory activity on FOXM1, FOXF1, FOXC1, FOXL1, FOXL2, FOXD1, FOXB2, FOXI2, and/or FOXN1 in step (X). Step (Y) above is characterized by measuring toxic activity of a test substance on young cells and toxic activity thereof on senescent cells, and selecting a test substance having no toxic activity on young cells and having toxic activity on senescent cells, and to a specific description thereof, the description of step (B) in <Method for screening a senolytic drug > can be applied as it is. In step (X) above, the inhibitory activity on FOXM1, FOXF1, FOXC1, FOXL1, FOXL2, FOXD1, FOXB2, FOXI2, and/or FOXN1 includes not only the activity of inhibiting the action of FOXM1, FOXF1, FOXC1, FOXL1, FOXL2, FOXD1, FOXB2, FOXI2, and/or FOXN1 but also the activity of suppressing the expression of FOXM1, FOXF1, FOXC1, FOXL1, FOXL2, FOXD1, FOXB2, FOXI2, and/or FOXN1.

Furthermore, the present invention also includes a method for screening a senolytic drug including: (X) a step of measuring enhancing activity of a test substance on FOXO1, FOXP1, FOXH1, FOXF2, FOXO4, FOXO3, FOXJ3, FOXQ1, FOXK2, FOXN3, FOXO6, FOXK1, FOXP4, FOXN2, and/or FOXJ2; and (Y) a step of measuring selective senolytic activity of a test substance determined to have enhancing activity on FOXO1, FOXP1, FOXH1, FOXF2, FOXO4, FOXO3, FOXJ3, FOXQ1, FOXK2, FOXN3, FOXO6, FOXK1, FOXP4, FOXN2, and/or FOXJ2 in step (X). Step (Y) above is characterized by measuring toxic activity of a test substance on young cells and toxic activity thereof on senescent cells, and selecting a test substance having no toxic activity on young cells and having toxic activity on senescent cells, and to a specific description thereof, the description of step (B) in <Method for screening a senolytic drug > can be applied as it is. In step (X) above, the enhancing activity on FOXO1, FOXP1, FOXH1, FOXF2, FOXO4, FOXO3, FOXJ3, FOXQ1, FOXK2, FOXN3, FOXO6, FOXK1, FOXP4, FOXN2, and/or FOXJ2 includes not only the activity of enhancing the action of FOXO 1, FOXP1, FOXH1, FOXF2, FOXO4, FOXO3, FOXJ3, FOXQ1, FOXK2, FOXN3, FOXO6, FOXK1, FOXP4, FOXN2, and/or FOXJ2 but also the activity of enhancing the expression of FOXO1, FOXP1, FOXH1, FOXF2, FOXO4, FOXO3, FOXJ3, FOXQ1, FOXK2, FOXN3, FOXO6, FOXK1, FOXP4, FOXN2, and/or FOXJ2.

EXAMPLES

The present invention will be specifically described with reference to the following Examples, but the present invention is not limited by the Examples.

1. Enhanced glycolysis and enhanced PGAM-Chkl binding in senescent cells

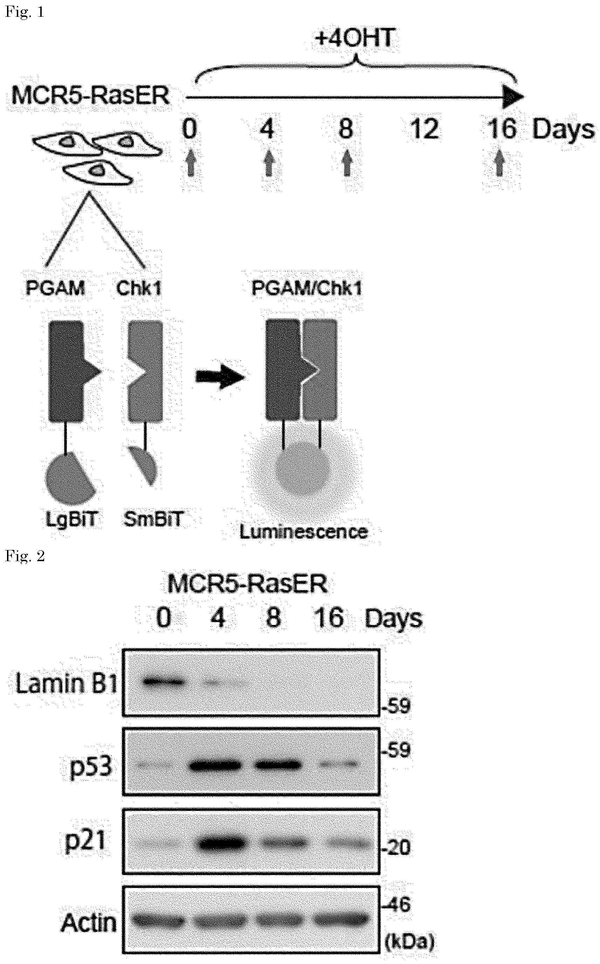

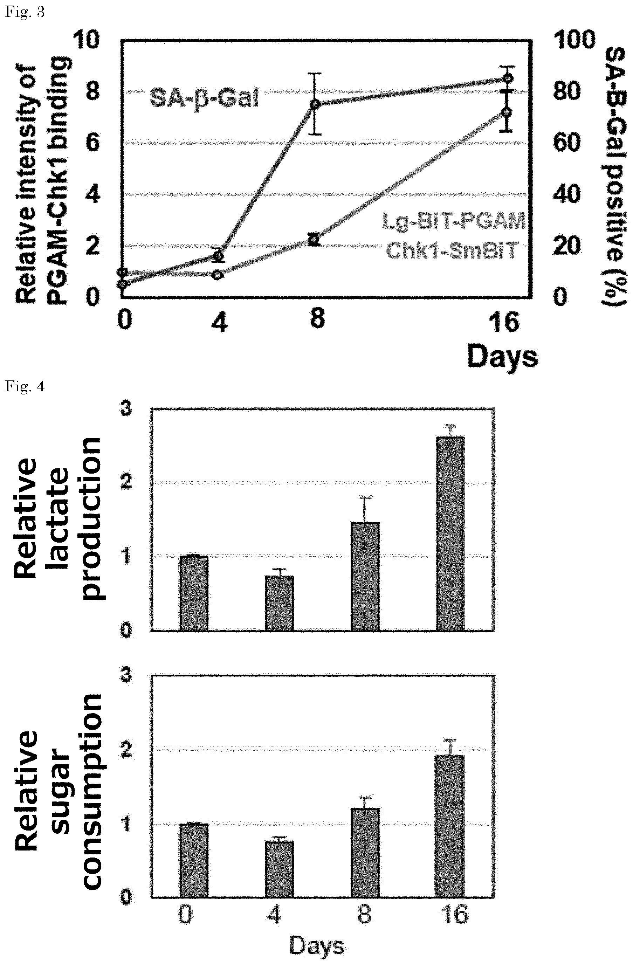

It was known that in normal cells, an oncogenic Ras mutation induces premature senescence (Serrano et al. Cell 1997, Vol. 88, Issue 5, 593-602). In addition, it was reported that glycolytic metabolism is enhanced in senescent cells (Goldstein and Trieman, 1975; Goldstein et al., 1982). Therefore, PGAM-Chkl interaction in senescent cells was investigated. Specifically, PGAM and Chkl kinase genes tagged with Nano-Bit tags were introduced into MCR5 cells (human fetal lung-derived normal diploid fibroblasts) to prepare a cell line capable of stable expression. The RasER gene was introduced into young MCR5 cells (within 1 month of continuous culture). The RasER protein is a fusion protein that can activate the Ras gene by administration of a 4-hydroxy tamoxifen agent (4OHT). As shown in Fig. 1, 4OHT was administered to MCR5-RasER cells, then the senescent phenotype was chronologically observed, and at the same time, PGAM-Chkl interaction was assessed by the Nano-Bit system. As shown in Fig. 2, senescent phenotypes (reduction in LaminB1, p53 accumulation, p21 accumulation) were observed from 4 days after 4OHT administration to these cells. Fig. 3 show the P interaction and SA-β Gal staining which is a marker for cellular senescence. SA-β Gal positive cells a marked increase in on day 8 to day 16. These cells were used to measure intracellular luminescence signals of Nano-Bit in each time points. In addition, the glycolytic metabolic efficiency in each timepoints was evaluated by measuring glucose consumption and lactate production in the medium. The lactate level is calculated as a value ($\mu$mol/mg/h) obtained by measuring the amount of lactate secreted into the medium per hour ($\mu$mol/h) and correcting the same by the total cell protein and is presented as a value relative to the actual value on day 0. The glucoseconsumption is calculated as a value ($\mu$mol/mg/h) obtained by measuring the amount of glucose decreased from the medium per hour ($\mu$mol/h) and correcting the same by the total cell protein and is presented as a value relative to the actual value on day 0. Glucose and lactate were measured by using a Glucose assay kit (BioVision) (lower diagram in Fig. 4) and a Lactate assay kit (BioVision) (upper diagram in Fig. 4), respectively.

As described above, in senescent cells, as shown in Fig. 3, a remarkable increase in the luciferase luminescence signal by Nano-Bit was observed (vertical axis), indicating PGAM-Chk1 interaction was enhanced. In addition, as shown in Fig. 4, it was found that both the lactate production and the glucose consumption efficiency were increased in senescent cells.

2. Senolysis by inhibiting PGAM-Chkl binding in senescent cells

The senolytic effects of nutlin 3a, which is known to be a PGAM-Chk1 binding inhibitor, and nutlin 3b, which is an optical isomer thereof, were examined. Nutlin 3a was purchased from Sigma-Aldrich, and nutlin 3b was purchased from Cayman Chemical.

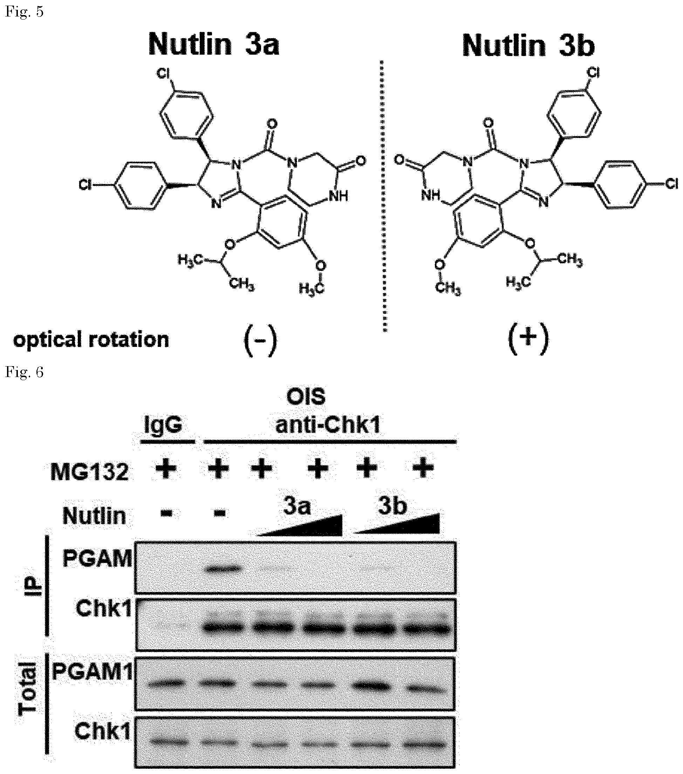

It is known that apoptosis of senescent cells is observed by using nutlin 3a, which is a PGAM-Chk1 binding inhibitor. That is, it is considered that inhibition of PGAM-Chkl binding induces apoptosis in senescent cells. However, nutlin 3a is known to activate p53 (Vassilev et al., 2004, Science 303, 844-848; hereinafter "Vassilev et al., 2004"), and negatively affects the proliferation and survival of both young cells and senescent cells and thus cannot be referred to as a senolytic drug. On the other hand, the present inventors have found a PGAM-Chkl binding inhibitor other than nutlin 3a. It is nutlin 3b, which is an optical isomer of nutlin 3a (Fig. 5). Nutlin 3b is a type of anticancer agent developed together with nutlin 3a as a chemical substance that inhibits the binding between p53 and Mdm2. However, it has been reported that there is a large gap between nutlin 3a and nutlin 3b in regard to inhibitory activity of p53-Mdm2 binding, and that the p53-Mdm2 binding inhibition by nutlin 3b is about 1/100 of that by nutlin 3a (Vassilev et al., 2004).

First, it was examined whether nutlin 3a and 3b inhibited PGAM-Chkl binding even in senescent cells, leading to decreased glycolytic metabolism. Specific methods and results will be described below.

Human primary fibroblasts, IMR90 cells, were obtained from the JCRB Cell Bank. An oncogenic Ras mutation was retrovirally introduced into young cells (within 1 month of continuous culture) of the IMR90 cells to induce cellular senescence. The obtained senescent cells were treated with a 20 $\mu$M or 40 $\mu$M concentration of nutlin 3a or 3b for 48 hours. After that, these cells were treated with MG132 for 6 hours, and cell lysates were prepared. The obtained cell lysates were immunoprecipitated with an anti-Chkl antibody. The resulting immunoprecipitate was used for Western blotting with an anti-PGAM antibody to evaluate PGAM-Chk1 binding. As a result, it was revealed that nutlin 3a and 3b can inhibit PGAM-Chkl interaction to the same extent in Ras-induced senescent cells (Fig. 6).

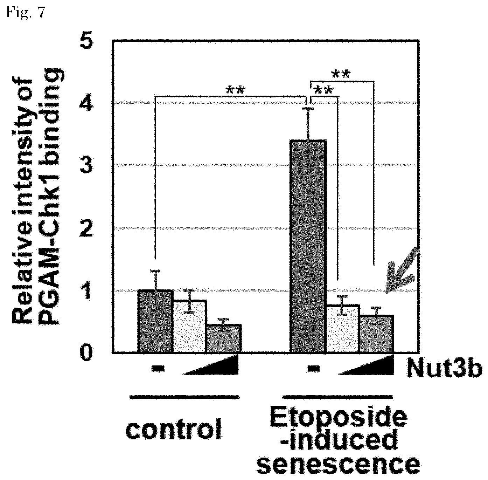

In addition, PGAM and Chkl kinase genes tagged with Nano-Bit tags were introduced into IMR90 cells to prepare a cell line capable of stable expression Young cells (within 1 month of continuous culture) of the IMR90 cells were treated with 100 uM etoposide for 48 hours to induce cellular senescence. The young cells and senescent cells of the IMR90 cells were treated with different concentrations of nutlin 3b (20 $\mu$M or 40 $\mu$M) for 48 hours, and intracellular luminescence signals of Nano-Bit in each cell were measured. As a result, the potentiation of PGAM-Chkl binding observed in senescent cells was suppressed by the nutlin 3b treatment in a dose-dependent manner (Fig. 7).

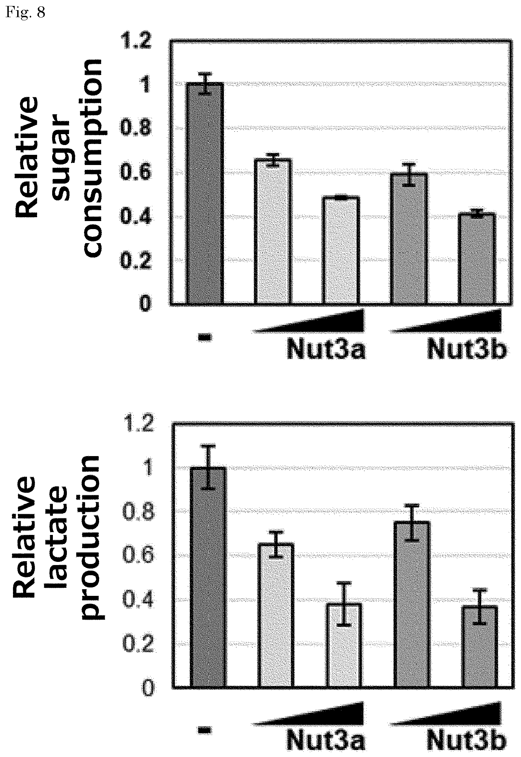

Next, young cells (within 1 month of continuous culture) of the IMR90 cells were treated with 100 $\mu$M etoposide for 48 hours to acquire senescent cells. The obtained senescent cells were treated with a 20 $\mu$M or 40 $\mu$M concentration of nutlin 3a or 3b for 48 hours. After the nutlin treatment, the medium was collected, and glucose consumption was evaluated and lactate production was measured. The lactate level is calculated as a value ($\mu$mol/mg/h) obtained by measuring the amount of lactate secreted into the medium per hour ($\mu$mol/h) and correcting the same by the total cell protein (mg) and is expressed as a value relative to the actual value on day 0. The glucose consumption is calculated as a value ($\mu$mol/mg/h) obtained by measuring the amount of glucose decreased from the medium per hour ($\mu$mol/h) and correcting the same by the total cell protein (mg) and is expressed as a value relative to the actual value on day 0. Glucose and lactate were measured by using a Glucose assay kit (BioVision) (upper diagram in Fig. 8) and a Lactate assay kit (BioVision) (lower diagram in Fig. 8), respectively. As a result, it was revealed that in senescent cells, both glucose consumption and lactate production were suppressed in a dose-dependent manner by nutlin 3a or 3b, confirming that glycolytic metabolism was suppressed.

As described above, first, it was also confirmed that the PGAM-Chkl binding inhibitors nutlin 3a and 3b inhibited PGAM-Chkl binding even in senescent cells, leading to decreased glycolytic metabolism. In addition, it was also revealed that a common metabolic property between senescent cells and cancer cells is PGAM-Chkl binding-dependent enhancement of glycolytic metabolism, and that inhibition of this binding induces decreased glycolytic metabolism in both cells.

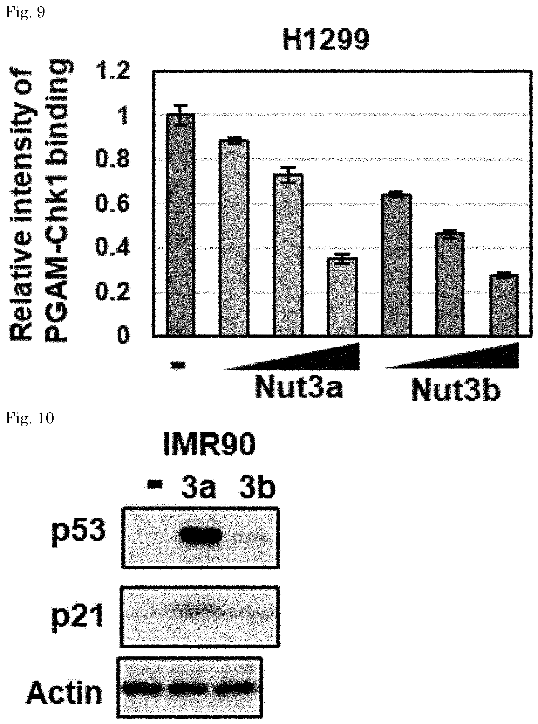

Next, it was found that nutlin 3a and nutlin 3b were almost equivalent in terms of the PGAM-Chkl binding inhibitory effect in cancer cells (Fig. 9). Specific test methods and results will be described below.

PGAM and Chkl kinase genes tagged with Nano-Bit tags were introduced into the cancer cell line H1299 to prepare a cell line capable of stable expression. The obtained cells were treated with a 40 $\mu$M concentration of nutlin 3a or 3b for 12, 24, and 48 hours. The treated cells were used to measure intracellular luciferase luminescence signals by Nano-Bit for each. As a result, the nutlin 3a or 3b treatment showed a treatment time-dependent decrease in luciferase luminescence signal by Nano-Bit (vertical axis), confirming suppression of PGAM-Chkl binding (Fig. 9).

However, p53 activation in young cells differed greatly between nutlin 3a and nutlin 3b, and the former achieved sufficient activation whereas the latter caused no activation at all (Fig. 10). This agrees with the finding in a previous report (Vassilev et al., 2004) that there is a large gap between nutlin 3a and nutlin 3b in terms of p53-Mdm2 binding inhibitory activity. Specific test methods and results will be described below.

Young cells (within 1 month of continuous culture) of the IMR90 cells were treated with 20 $\mu$M nutlin 3a or 3b for 48 hours. After the treatment, proteins were extracted from the cells to carry out Western blotted. The protein levels of p53 and the downstream factor thereof p21 were markedly increased by nutlin 3a, whereas the protein levels were almost unchanged by nutlin 3b (Fig. 10).

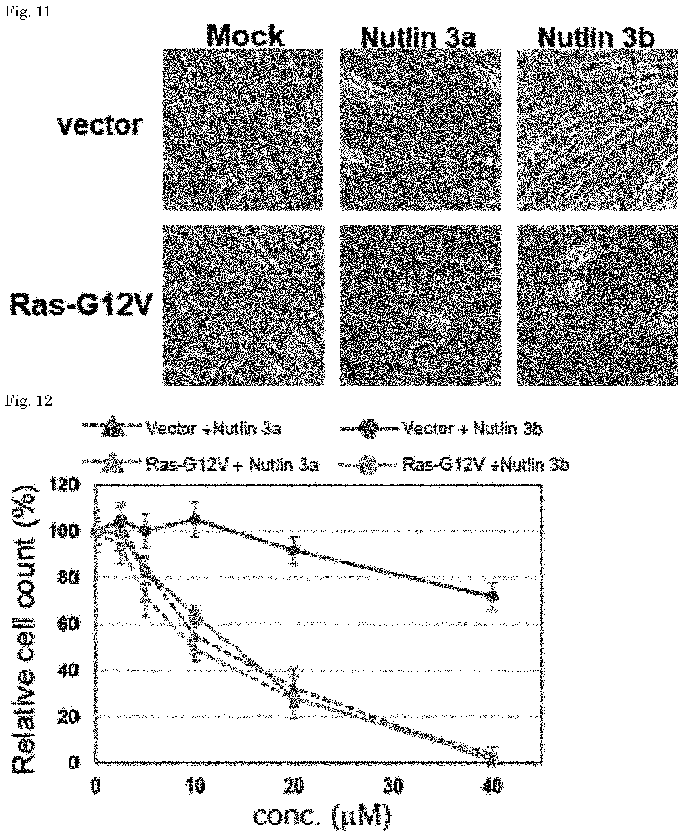

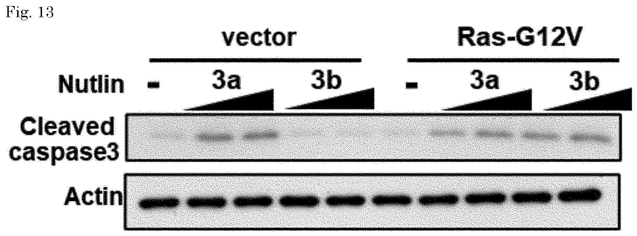

Next, the effects of nutlin 3a and 3b on young cells and senescent cells were compared. As a result, it was found that unlike nutlin 3a, nutlin 3b induces selective apoptosis (senolysis) of cultured senescent cells whereas showing no toxicity to young cells (Fig. 11). In addition, the senolytic effect of nutlin 3b was dose-dependent (Fig. 12), and Western blotting of caspase 3 fragmentation confirmed apoptosis of senescent cells (Fig. 13). Specific test methods and results will be described below.

Ras gene with oncogenic mutation was introduced into young cells (within 1 month of continuous culture) of IMR90 cells by retroviral infection to acquire senescent cells. The young cells and the senescent cells of IMR90 were both treated with 40 $\mu$M nutlin 3a or 40 $\mu$M nutlin 3b for 72 hours. The control received only DMSO treatment. Representative images of cells observed under a microscope are shown (Fig. 11). In case of nutlin 3a, cell death and cell number decrease were observed in both the young cells and the senescent cells, whereas with nutlin 3b treatment, a senolysis phenomenon (young cells are normal, but senescent cells selectively die) was observed.

Next, the above young cells and senescent cells were both treated with different concentrations of nutlin 3b for 96 hours, and evaluated for cell death. A remarkable senolytic effect was confirmed with a 20 or 40 $\mu$M concentration of nutlin 3b (Fig. 12).

Ras gene with oncogenic mutation was introduced into young cells (within 1 month of continuous culture) of IMR90 cells by retroviral infection to acquire stress senescent cells. The young cells and the senescent cells were both treated with different concentrations of nutlin 3b for 72 hours, and evaluated for cell death by Western blotting of fragmented caspase-3. In the young cells, the nutlin 3b treatment did not cause caspase-3 fragmentation. On the other hand, in the senescent cells, dose-dependent fragmentation of caspase 3 was observed by the nutlin 3b treatment (Fig. 13).

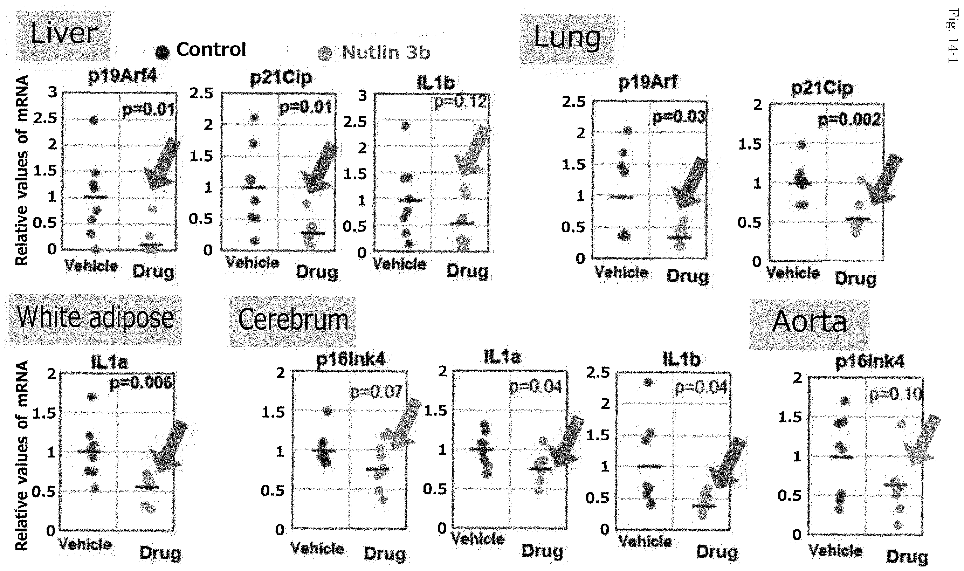

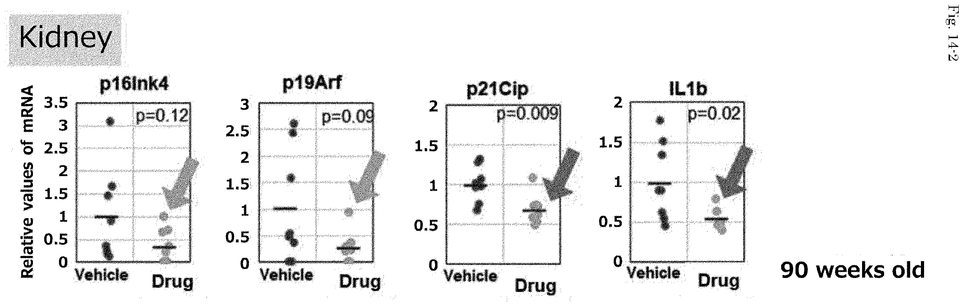

Next, the effect of nutlin 3b administration to aged mice was examined. Specifically, 16 aged C57BL/6 mice at 90 weeks of age were provided. The mice were divided into two groups (control: 8 mice, agent group: 8 mice), and intraperitoneal injection administration once a week was continued for 2 months. The control group was injected with physiological saline, and the agent group was given nutlin 3b at a concentration of 11.63 mg/kg. One week after the end of the last administration, all mice were dissected, and RNA was extracted from each organ. Senescence markers and inflammation markers were evaluated by RT-PCR. Results thereof are shown in Figs. 14-1 and 14-2. As shown in Figs. 14-1 and 14-2, it was confirmed that the senescence markers and the inflammatory markers were reduced in the agent administration group (nutlin 3b). As with the prior drug ABT263 (Chang et al., Nat. Med., 2016), it was confirmed that the nutlin 3b administration reduced various senescence markers.


3. Mechanism analysis of PGAM-Chkl kinase binding


Oncogenic mutated Ras is one of the oldest oncogenes discovered, and has a very high frequency in pancreatic cancer, lung cancer, and large intestine cancer (90%, 30%, and 50%, respectively). The present inventors have found that PGAM-Chkl kinase binding was induced in cancer cells expressing an oncogenic Ras mutation to maintain enhanced glycolytic metabolism (Warburg effect) in cancer (Mikawa et al., 2020, iScience 23, 101306; hereinafter "Mikawa et al., 2020"). Chkl kinase is a serine-threonine protein kinase, and is a checkpoint kinase that plays a role in activating a DNA damage checkpoint in the G2 phase of the cell cycle. Based on the above findings, it was found that Chkl also plays an important role in glycolytic metabolism (Mikawa et al., 2020). PGAM-Chk1 kinase binding was able to be observed in cancer cells expressing an oncogenic Ras mutation, but not able to be observed in normal cells not expressing an oncogenic Ras mutation (Mikawa et al., 2020).

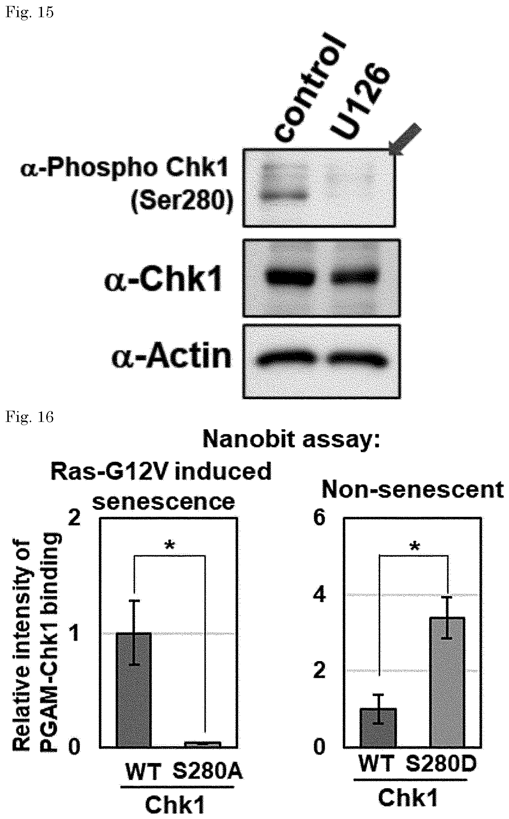

A more detailed investigation of the signal pathway by an oncogenic Ras mutation revealed that MEK kinase and RSK kinase present downstream thereof are important (Mikawa et al., 2020). When each inhibitor (U126 and BI-D1870) was administered to cancer cells, PGAM-ChkI kinase binding was inhibited, and glycolytic metabolism decreased (Mikawa et al., 2020). RSK kinase was already known to directly control the phosphorylation of the S280 residue of ChkI kinase (Li et al., 2012, Mol Biol Cell 23, 1582-1592). The present inventors confirmed that the RSK kinase inhibitor also suppressed the S280 phosphorylation of ChkI kinase in H1299 cells (Fig. 15). Specifically, cancer cell line H1299 cells were treated with the MEK kinase inhibitor (U126) at a concentration of 10 μM for 48 hours. The treated cells were collected, and proteins were extracted and then Western blotted. It was confirmed that the S280 phosphorylation of ChkI kinase was suppressed although the protein level of ChkI kinase was not changed. In addition, the present inventors prepared S280A mutants of ChkI kinase. It was found that this non-phosphorylatable mutant (Chk1-S280A) inhibited PGAM-ChkI kinase binding in senescent cells (left in Fig. 16). In contrast, it was found that the phosphorylation-mimetic mutant (Chk1-S280D) enhanced PGAM-ChkI kinase binding in young cells (right in Fig. 16). Specifically, PGAM and ChkI kinase genes tagged with NanoBit tags were introduced into IMR90 cells to prepare a cell line capable of stable expression. At this time, three types of Chk1, i.e., wild-type ChkI and S280A mutant ChkI or S280D mutant Chk1, were each introduced into the cells. An oncogenic mutated Ras gene was introduced into these cells by retroviral infection to acquire stress senescent cells. The obtained senescent cells were used to measure intracellular luciferase luminescence signals by Nano-Bit for each. As a result, the S280A mutation showed a decrease in luciferase luminescence signal by Nano-Bit (vertical axis), confirming suppression of PGAM-ChkI binding (left in Fig. 16). Even in young cells into which no oncogenic mutated Ras gene had been introduced, the S280D mutation showed enhancement of the luciferase luminescence signal by Nano-Bit (right in Fig. 16). From the above, it was revealed that the S280 phosphorylation of ChkI kinase by RSK kinase, which is a downstream signal kinase of the oncogenic Ras mutation, is important for PGAM-ChkI kinase binding.

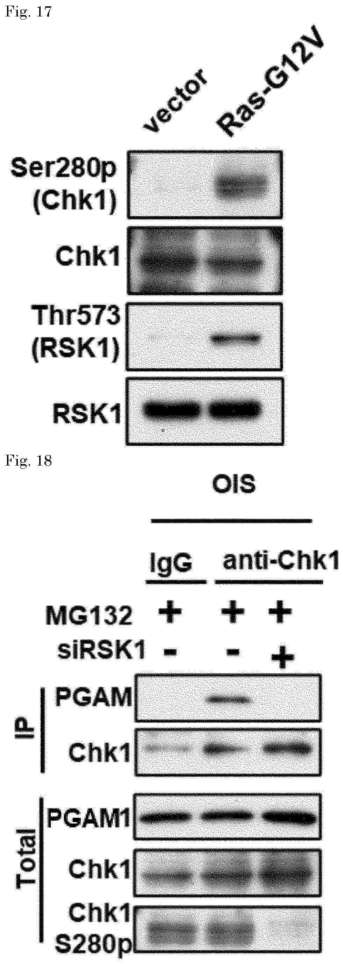

Furthermore, it was found that RSK1-Thr573 phosphorylation was enhanced and Chk1-Ser280 phosphorylation was also enhanced in senescent cells made by the oncogenic Ras mutation (Fig. 17). In addition, it was found that RSK siRNA treatment inhibited PGAM-ChkI binding in senescent cells (Fig. 18). Specific test methods and results will be described below.

An oncogenic mutated Ras gene was introduced into young cells (within 1 month of continuous culture) of IMR90 cells by retroviral infection to acquire stress senescent cells. The obtained young cells and senescent cells were collected, and proteins were extracted and then Western blotted. In the senescent cells, the S280 phosphorylation of ChkI kinase and the Thr573 phosphorylation of RSK1 kinase were enhanced, and ChkI and RSK1 kinase were activated (Fig. 17).

Next, in the same manner as above, IMR90 cells were used to prepare stress-induced senescent cells by introducing an oncogenic mutated Ras gene. The senescent cells were transfected with an RSK1 siRNA (SEQ ID NO: 7) or a control to knock down RSK1. The resulting cells were collected, and proteins were extracted. The resulting protein extract was immunoprecipitated with an anti-ChkI antibody. The resulting immunoprecipitate was used for Western blotting with an anti-PGAM antibody to evaluate PGAM-Chk1 binding. As a result, it was revealed that in the senescent cells, the knockdown of RSK1 decreased the S280 phosphorylation of ChkI kinase, enabling inhibition of PGAM-ChkI (Fig. 18).

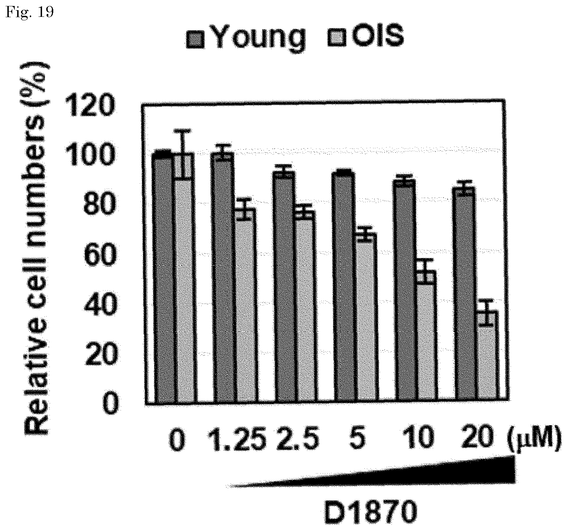

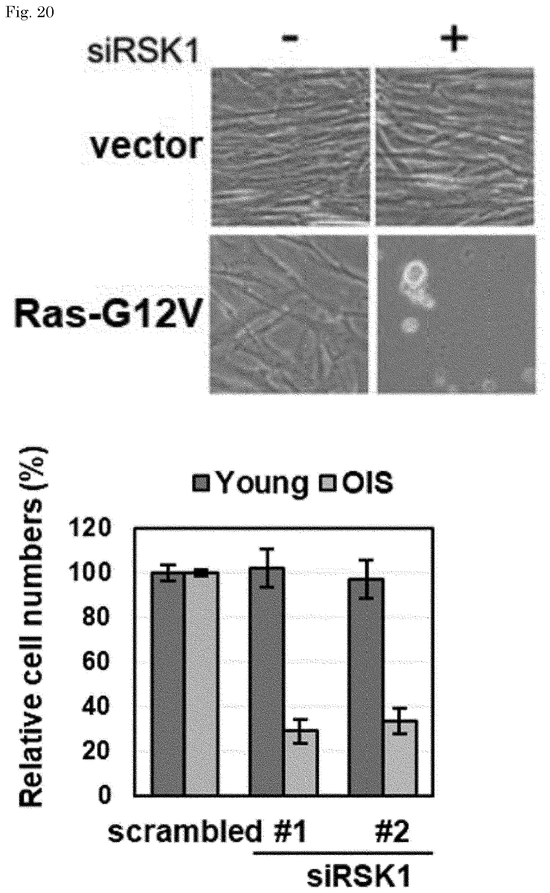

Next, the senolytic effect of RSK kinase inhibition was verified. A senolytic effect was observed with the RSK kinase inhibitor BI D1870 (Fig. 19) and knockdown by the RSK1 siRNA (Fig. 20) as with nutlin 3b. The senolysis by the RSK inhibitor was dose-dependent (Fig. 19), and the senolytic effect of the siRNA was comparable to or more efficient than the former (Fig. 20). Specific test methods and results will be described below.

An oncogenic mutated Ras gene was introduced into young cells (within 1 month of continuous culture) of IMR90 cells by retroviral infection to acquire stress-induced senescent cells. The young cells and the senescent cells were both treated with different concentrations of the RSK kinase inhibitor BI-D1870 for 96 hours to evaluate cell death. A remarkable senolytic effect was confirmed with a 10 μM or 20 μM concentration of BI-D1870 (Fig. 19).

An oncogenic mutated Ras gene was introduced into young cells (within 1 month of continuous culture) of IMR90 cells by retroviral infection to acquire stress-induced senescent cells. The young cells and the senescent cells of IMR90 were both transfected with an RSK1 siRNA (two types of siRNA #1 (SEQ ID NO: 7) or #2 (SEQ ID NO: 8) having different target sequences) or a control to knock down RSK1. Representative images of cells observed under a microscope are shown (upper diagram in Fig. 20). With the knockdown of RSK1, a senolysis phenomenon (young cells are normal, but senescent cells selectively die) was observed (lower diagram in Fig. 20).

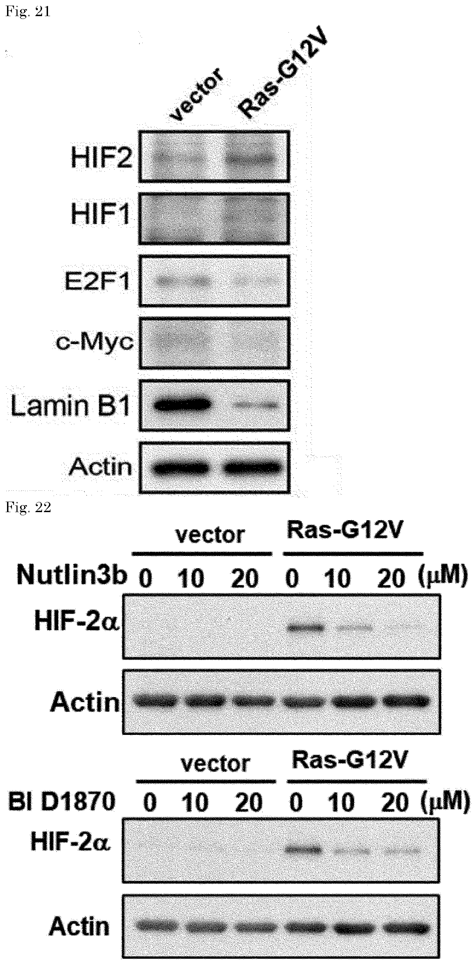

An oncogenic Ras was introduced into young cells (within 1 month of continuous culture) of IMR90 cells to induce senescent cells (OIS). Expression of transcription factors (HIF-2α, HIF-1α, E2F1, c-Myc) involved in glycolysis regulation in young cells and senescent cells of IMR90 was compared by Western blotting. A marked decrease in Lamin B1, which is an indicator of senescence, was confirmed in the senescent cells. Results thereof are shown in Fig. 21. As shown in Fig. 21, it was found that HIF-2α markedly accumulated in the senescent cells.

In addition, the transcription factor HIF-2α was Western blotted in both young cells and senescent cells treated with the different concentrations of nutlin 3b or the RSK kinase inhibitor BI-D1870 for 96 hours. As a result, it was observed that the HIF-2α protein decreased in the nutlin 3b-treated group and the BI-D1870-treated group (upper diagram and lower diagram in Fig. 22).

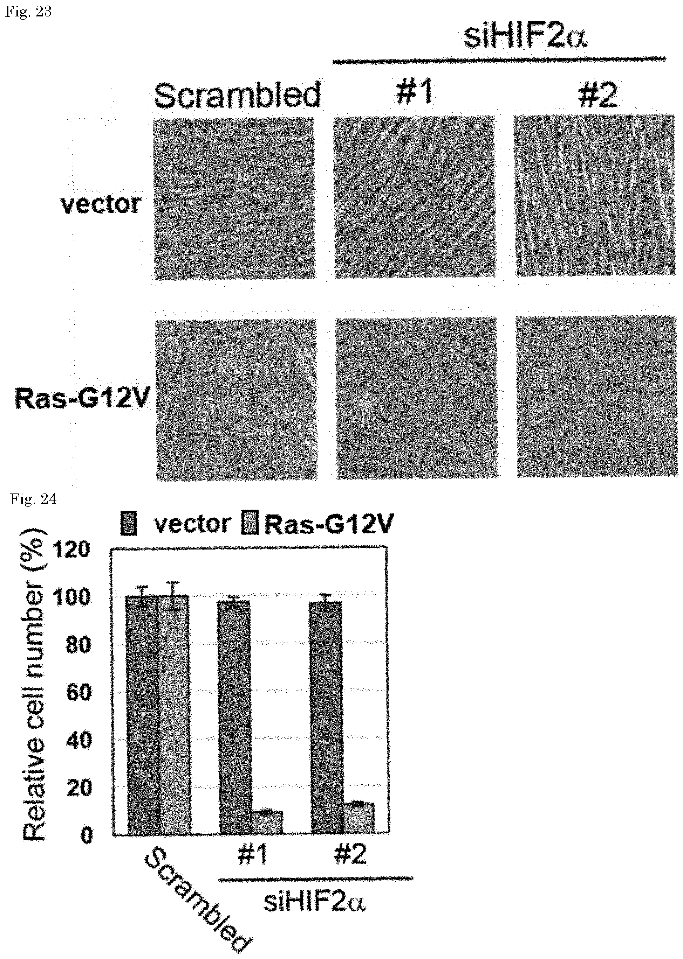

An oncogenic Ras was introduced into young cells (within 1 month of continuous culture) of IMR90 cells to induce senescent cells (OIS). The young cells and the senescent cells of IMR90 were transfected with two HIF-2α-specific siRNAs (SEQ ID NO: 9 and SEQ ID NO: 10) having different target sequences to knock down HIF-2α. After that, when cell death was evaluated, selective cell death (senolysis) was observed in the senescent cells in which HIF-2α was knocked down by using the two HIF-2α-specific siRNAs having different target sequences. Results thereof are shown in Figs. 23 and 24.

4. Analysis of downstream transcription factors of PGAM1-Chk1 pathway

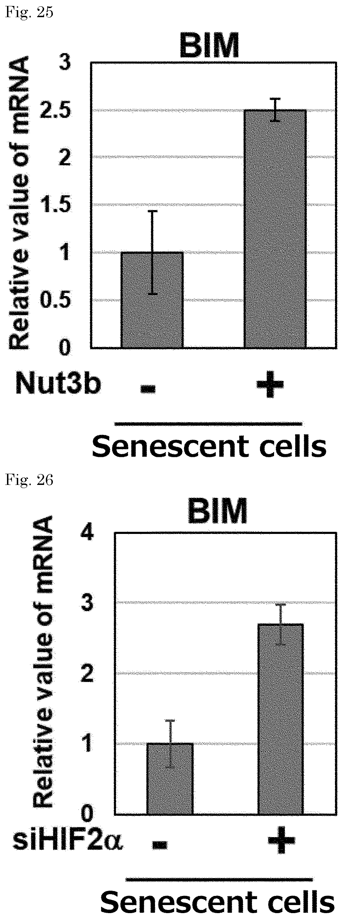

An oncogenic Ras was introduced into young cells (within 1 month of continuous culture) of IMR90 cells to induce senescent cells (OIS). The senescent cells were treated with nutlin 3b for 48 hours, and the expression of the BIM gene, which is an apoptosis-inducing gene, was analyzed (Fig. 25). As shown in Fig. 25, it was confirmed that the nutlin 3b treatment of the senescent cells increased the expression of the apoptosis-inducing gene BIM.

An oncogenic Ras was introduced into young cells (within 1 month of continuous culture) of IMR90 cells to induce senescent cells (OIS). The senescent cells were transfected with an HIF-2α-specific siRNA (SEQ ID NO: 9) to knock down HIF-2α. After that, the expression of the BIM gene, which is an apoptosis-inducing gene, was analyzed (Fig. 26). As shown in Fig. 26, it was confirmed that the HIF-2α knockdown in the senescent cells increased the expression of the apoptosis-inducing gene BIM.

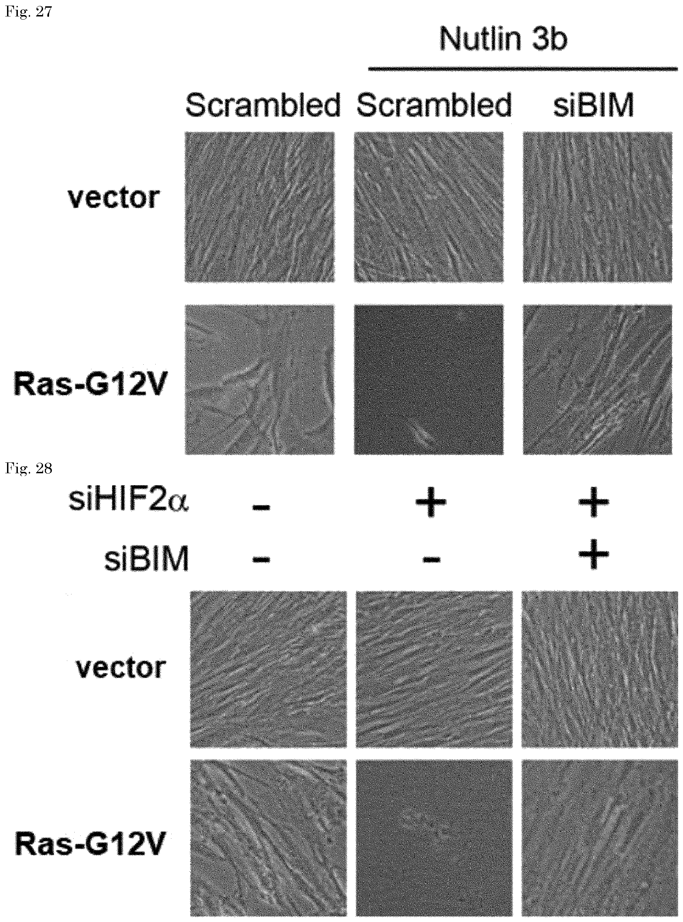

An oncogenic Ras was introduced into young cells (within 1 month of continuous culture) of IMR90 cells to induce senescent cells (OIS). The young cells and the senescent cells of IMR90 were transfected with a BIM-specific siRNA to knock down BIM. After that, nutlin 3b treatment was carried out for 96 hours, and cell death was confirmed (Fig. 27). As shown in Fig. 27, it was found that senolysis by nutlin 3b treatment is rescued by BIM knockdown.

An oncogenic Ras was introduced into young cells (within 1 month of continuous culture) of IMR90 cells to induce senescent cells (OIS). In the young cells and the senescent cells of IMR90, HIF-2α alone or both HIF-2α and BIM were knocked down by using their respective specific siRNAs (SEQ ID NO: 9, SEQ ID NO: 10), and cell death was confirmed (Fig. 28). As shown in Fig. 28, it was found that senolysis by HIF-2α knockdown is rescued by BIM knockdown.

5. Senolytic drug screening

The above data was comprehensively considered, and it was speculated that if PGAM-Chk1 binding is used as an indicator, it would be possible to identify a more powerful senolytic drug. For this purpose, PGAM-Chkl binding inhibition HTP (high-throughput) screening using a Cell-base NanoBiT system with visualization of PGAM-Chkl binding as an indicator was carried out. Specifically, the screening was carried out by the following method. In NanoBiT, a large subunit and a small subunit of a photoprotein prepared based on a luciferase are attached as tags to PGAM and Chk1, respectively. These tags have structures that can bind to each other. Each subunit itself does not emit light, but the binding between PGAM and Chk1 causes the large subunit and the small subunit to associate to form a complete photoprotein, thereby emitting a luminescence signal. By measuring the intensity of the luminescence signal at this time, the binding level can be measured with high sensitivity and high quantitativity. At this time, the luminescence signal decreases in the presence of a substance that inhibits the binding of PGAM and Chk1, such as nutlin. Therefore, it is possible to screen a drug library for a compound that inhibits the binding of PGAM and Chk1, with the above decrease in luminescence signal as an indicator.

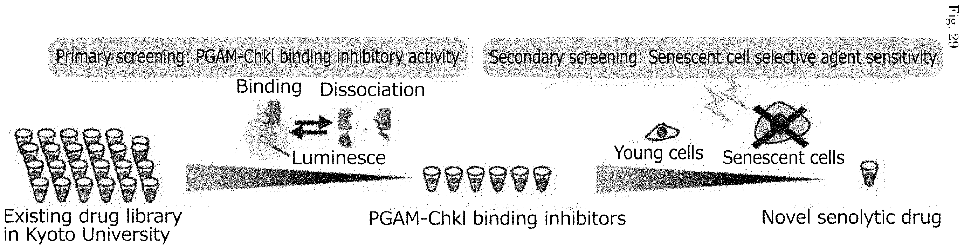

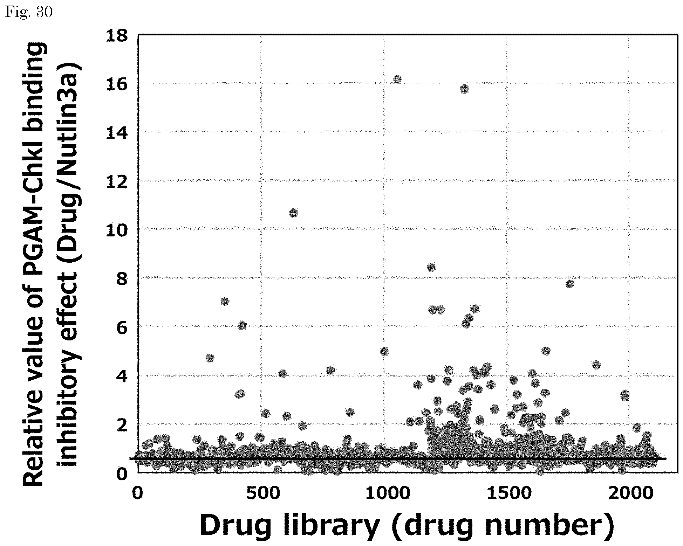

By using this PGAM-Chkl binding visualization Cell-base NanoBiT system, with the support of the Kyoto University Medical Research Support Center, PGAM-Chkl binding inhibition HTP screening was carried out on a library of existing drugs (more than 2300) having established safety evaluation, and slightly more than about 200 binding inhibitory candidate drugs were acquired in primary screening (Fig. 29 and Fig. 30).

Furthermore, in secondary screening, senolytic activity on normal cells was used as an indicator. About 40 senolytic drugs having mechanisms different from that of ABT263, which has senolytic activity, were found (Fig. 29). Specific test methods and results will be described below.

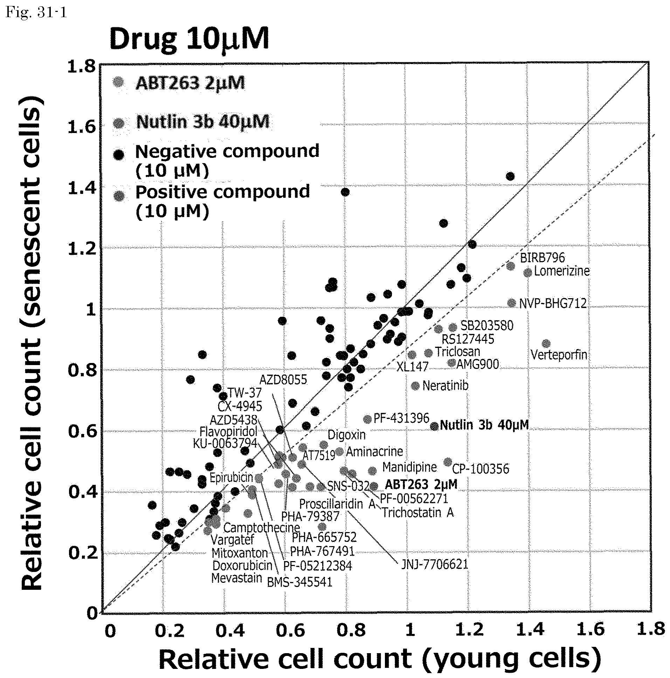

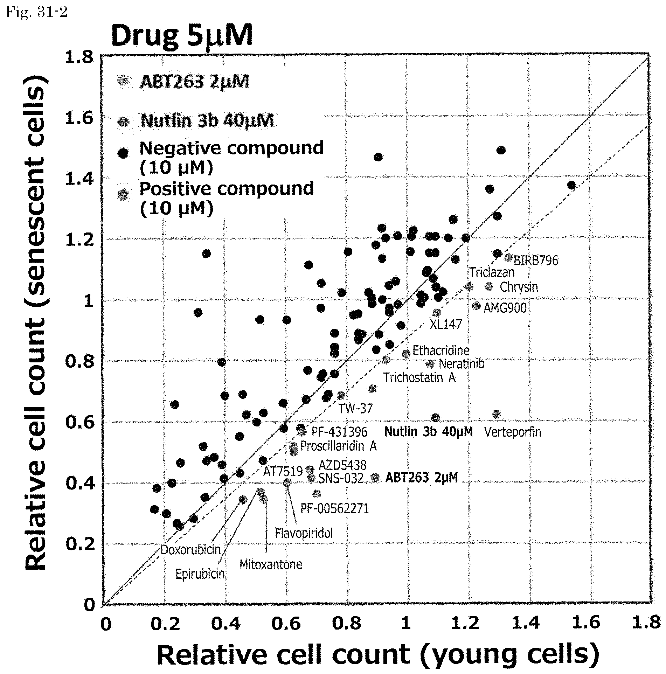

In the secondary screening, the agent sensitivity of young IMR90 cells and senescent IMR90 cells was compared. The senescent IMR90 cells were prepared by inducing an oncogenic Ras mutation. The young cells and the senescent cells were each seeded in a 96-well plate, and two days later, the candidate drugs obtained from the primary screening were treated at two concentrations of 5 μM and 10 μM. Surviving cells after 72 hours were fixed with formalin and stained with crystal violet. After that, crystal violet in each well was extracted with 2% Triton X100 and quantified (Fig. 31-1 and Fig. 31-2). Young cells and senescent cells were each treated with a solvent (DMSO), and the resulting cells were used as a negative control, and the survival rate compared to the negative control was calculated by the following expression. Furthermore, the agent sensitivity of the young cells and the senescent cells was compared and evaluated as senolytic activity (young cell viability/senescent cell viability). The criterion of positivity was a senolytic activity of 1.14 or more.

$$\text{Viability (\%)} = (\text{Sample/negative subject}) \times 100$$

Senolytic activity = young cell viability/senescent cell viability

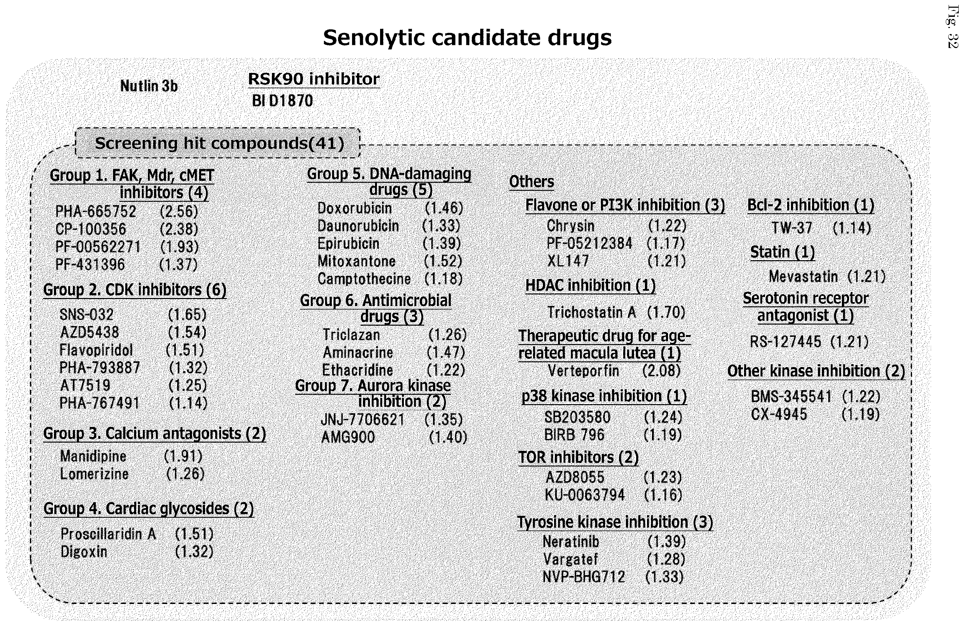

As described above, at present, the present inventors have successfully identified more than 40 senolytic drugs, including nutlin 3b and the RSK kinase inhibitor BI-D1870, and agents acquired in the secondary screening, as summarized in Fig. 32. These novel senolytic drugs acquired in the secondary screening were able to be classified into approximately 7 groups by functional classification.

6. Examination of effect of nutlin 3b administration to aged mice

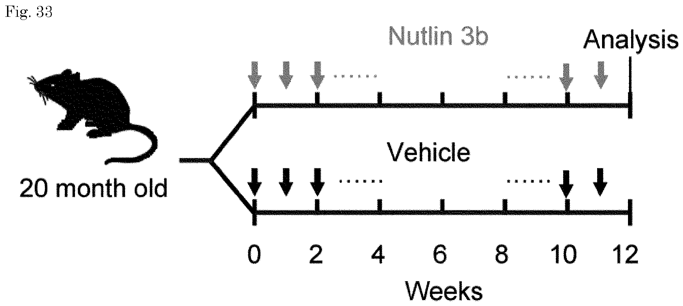

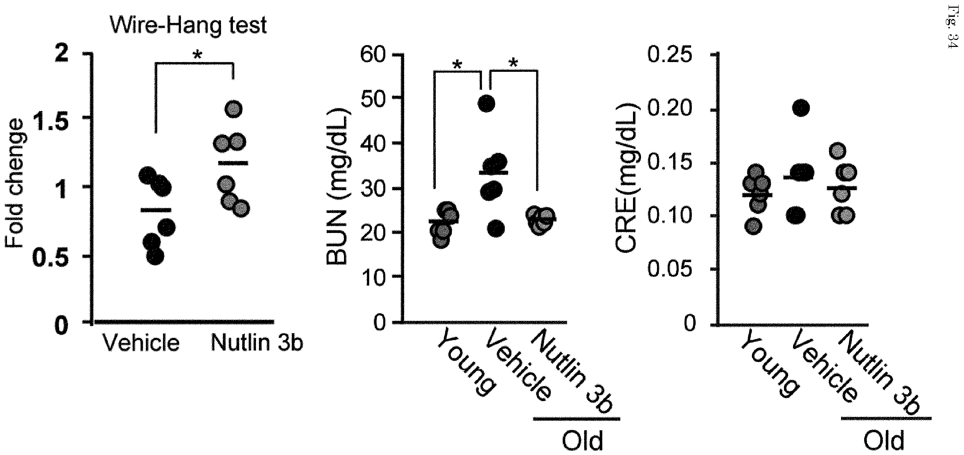

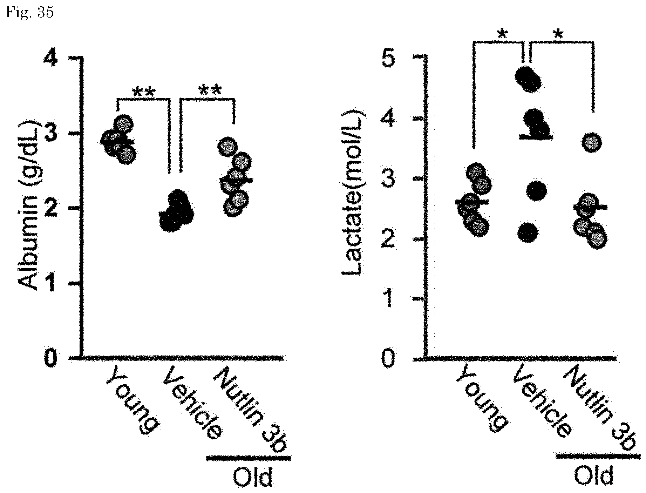

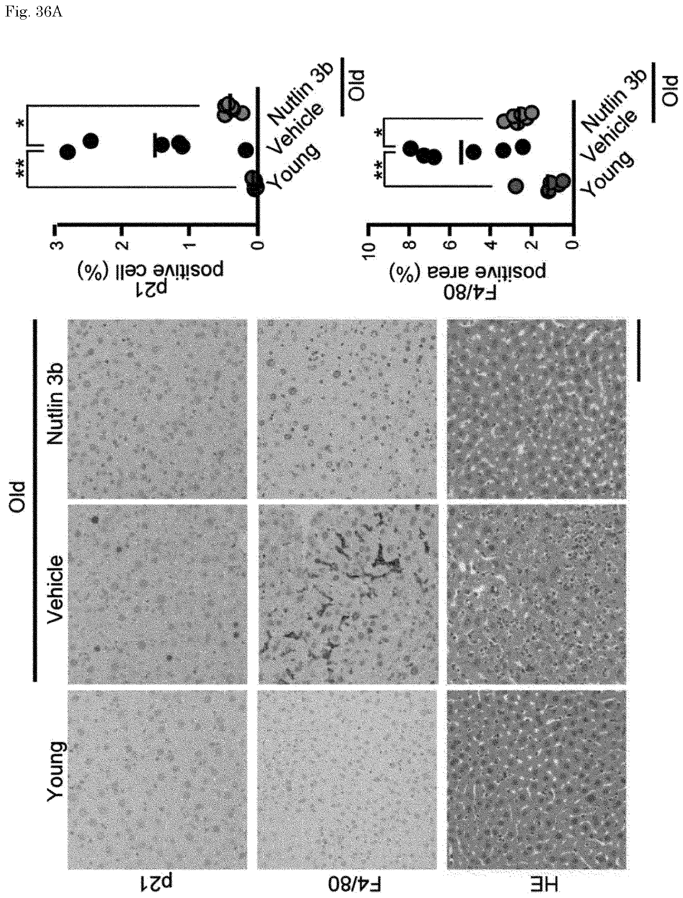

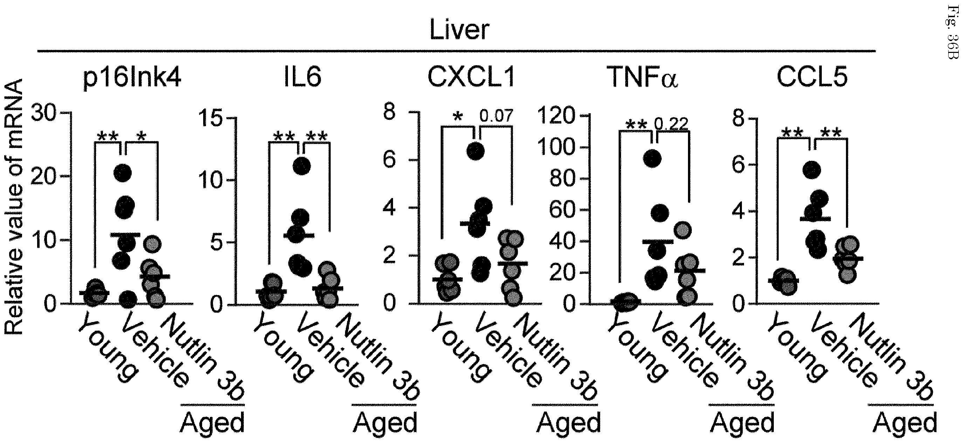

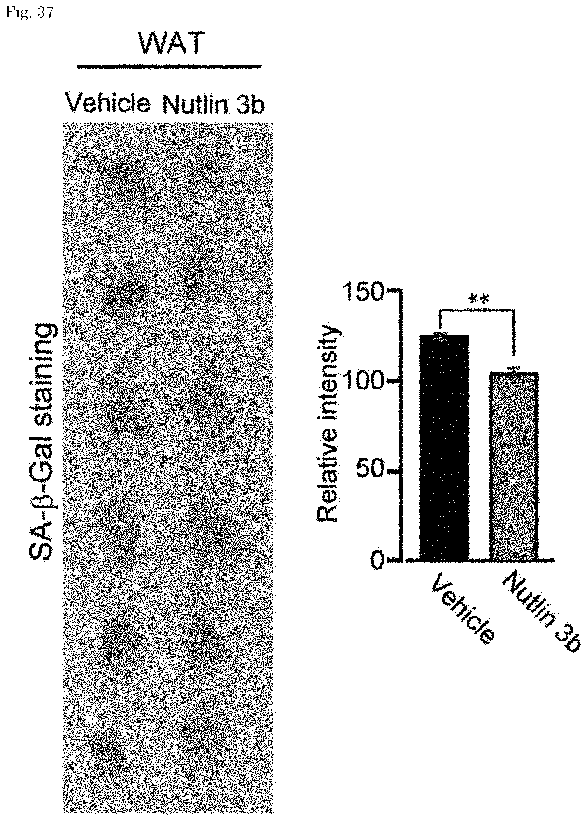

The in vivo effect of nutlin 3b in aged mice was evaluated in more detail. The test schedule is shown in Fig. 33. Twenty-month-old C57BL/6 mice (Jackson Laboratory Japan) were divided into two groups, and the nutlin 3b administration group was intraperitoneally given nutlin 3b suspended in a solvent (PBS:PEG400 = 50:50) at 11.62 mg/kg once a week for 3 months. The vehicle group was given a solvent alone on the same schedule. As a result, some improvements in physical condition were observed in the nutlin 3b administration group. In addition to muscle strength (wire hang test) and the renal function markers plasma BUN and plasma creatinine, the liver function markers plasma albumin level and lactate level were improved by the nutlin 3b administration (Figs. 34 and 35). Histochemical analysis of the liver revealed that p21-positive cells accumulating in aged mice significantly decreased in nutlin 3b-treated aged mice (Fig. 36A). F4/F80-positive cells, indicative of macrophage infiltration in aged tissue, were also reduced by the nutlin 3b treatment, which is consistent with reductions in several SASP factors (IL6, CXCL1, TNF$\alpha$, and CCL5). In addition, when adipose tissue of each group was stained with senescence-associated $\beta$-galactosidase (SA $\beta$-Gal), which is a cellular senescence marker, the degree of cellular senescence was improved in the nutlin 3b administration group (Fig. 37).

7. Analysis of downstream transcription factors of PGAM1-Chk1 pathway (control of HIF-2$\alpha$ by Chkl)

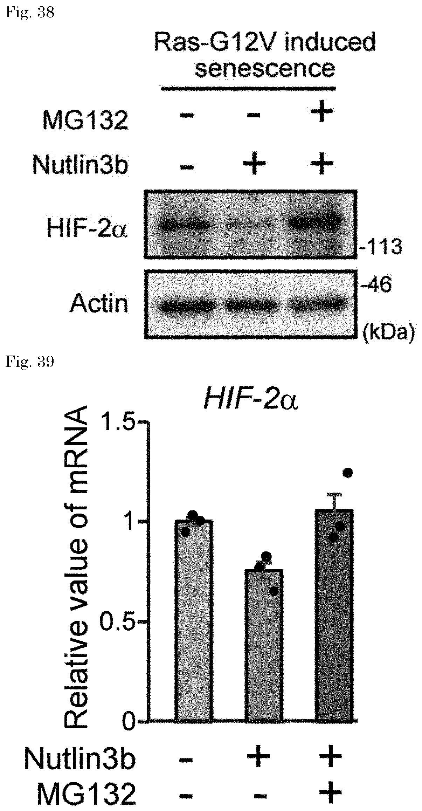

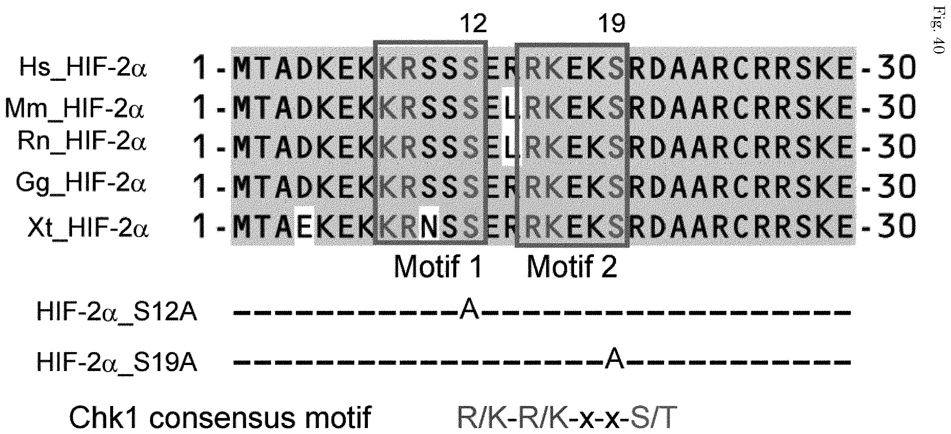

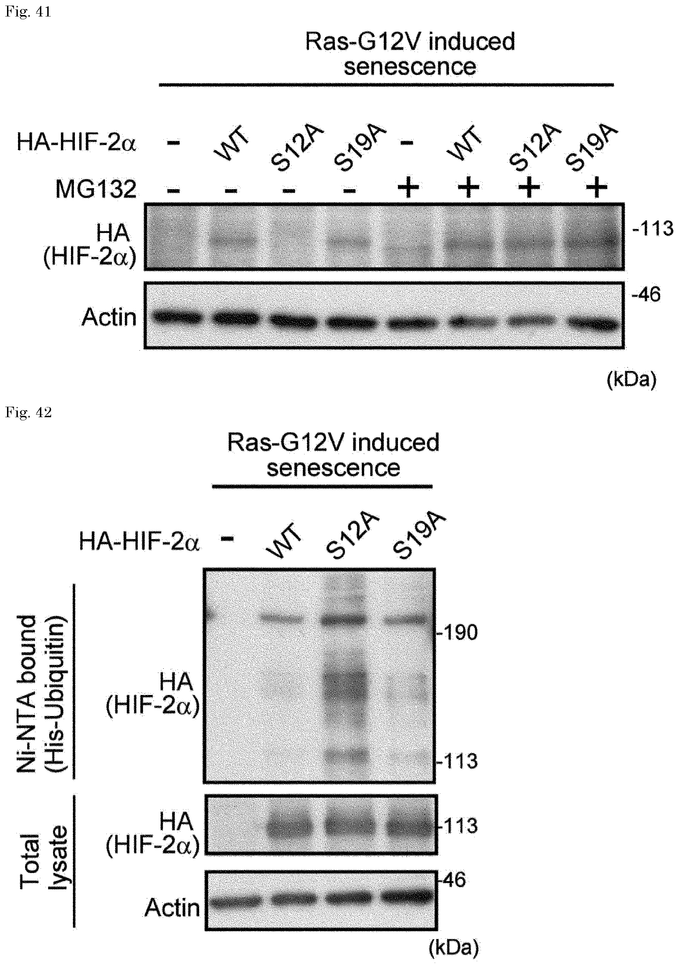

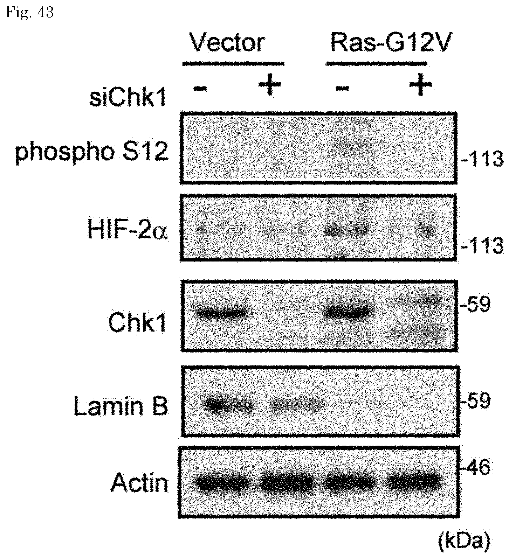

An oncogenic Ras was introduced into young cells (within 1 month of continuous culture) of IMR90 cells to induce senescent cells (OIS). Treatment of these senescent cells with nutlin 3b suppressed HIF-2$\alpha$ protein expression, but treatment with the proteasome inhibitor MG132 remarkably restored the HIF-2$\alpha$ protein level (Fig. 38). However, the influence thereof on an mRNA was relatively small (Fig. 39), suggesting a possible involvement of ubiquitination of HIF-2$\alpha$ during the nutlin 3b treatment. It is known that ubiquitination is induced by phosphorylation in the vicinity of a ubiquitination site (Hagai et al., 2012). The consensus sequences (K/R-K/R-x-x-S/T) for phosphorylation by Chkl located at the N-terminus of HIF-2$\alpha$ were conserved, and these were named Motif1 (including Ser12 residues) and Motif2 (including Ser19 residues) (Fig. 40, SEQ ID NOs: 77 to 81). Among these, it has been reported that Ser12 of HIF-2$\alpha$ is also phosphorylated in other cell types according to a global phosphorylated protein (https://www.phosphosite.org (Hornbeck et al., 2015) database. The S12A mutant of HIF-2$\alpha$ was very vulnerable to ubiquitination in senescent cells, but the S19A mutant was not (Figs. 41 and 42). Consistently, phosphorylation of Ser12 residues was detected in senescent cells in a Chkl-dependent manner (Fig. 43). These data suggest that HIF-2$\alpha$ is a senescence target of PGAM-Chkl interaction.

8. Examination of physiological significance of enhanced glycolysis in senescent cells

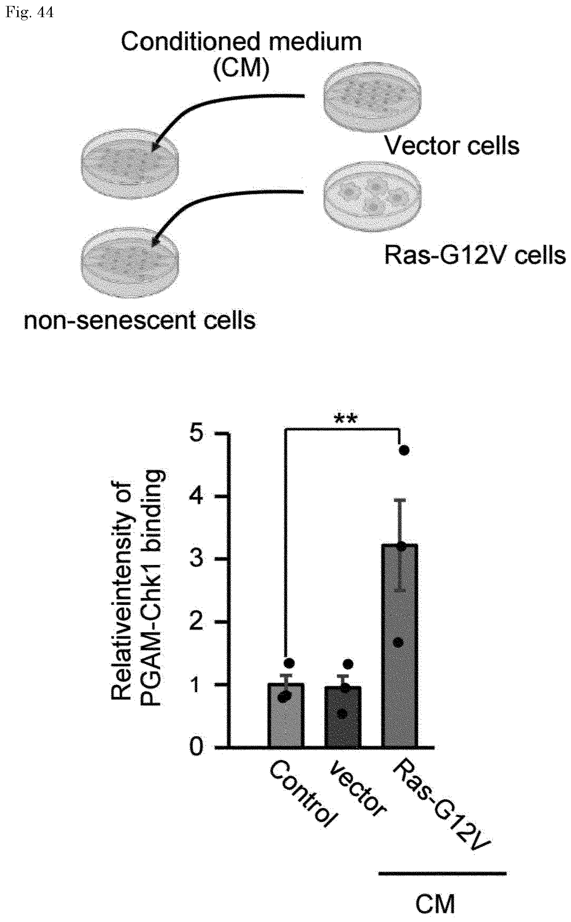

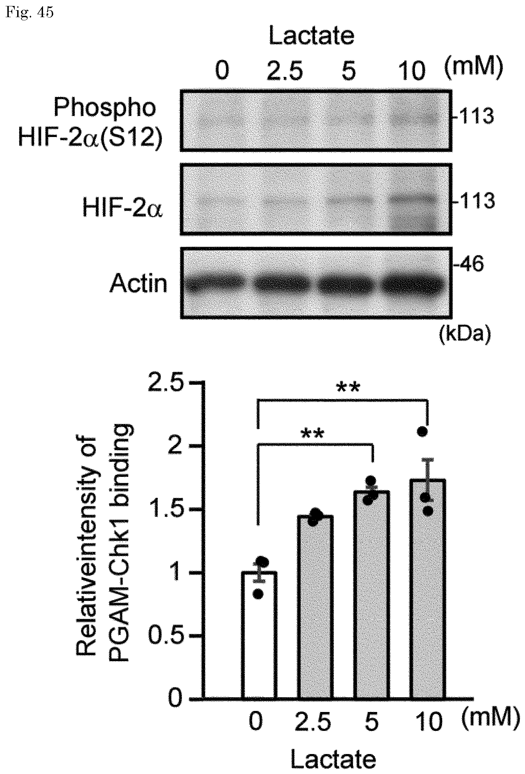

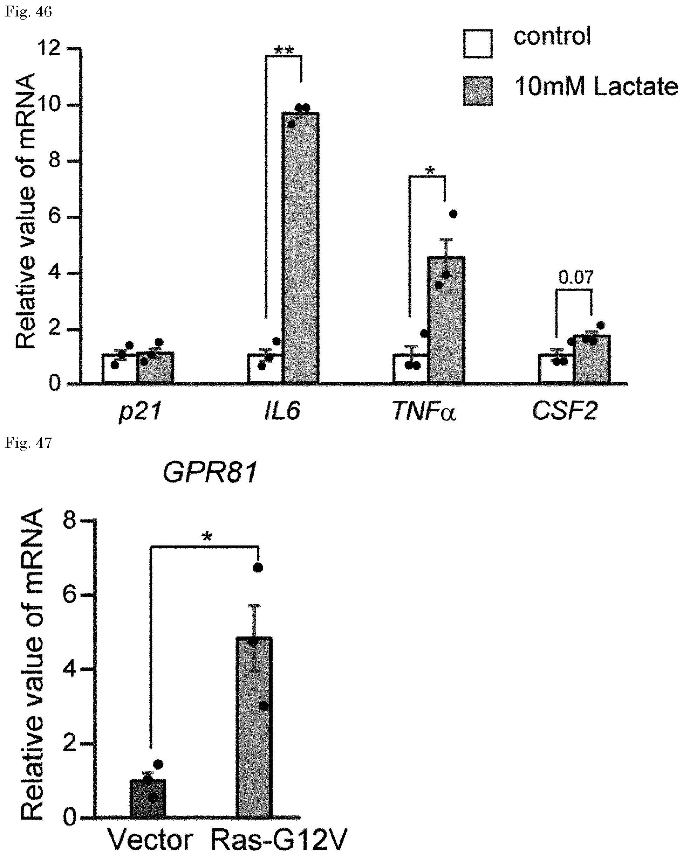

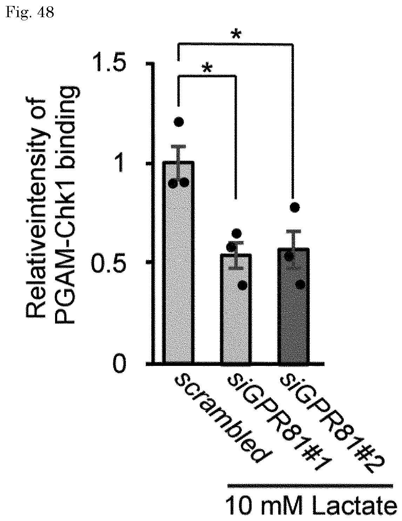

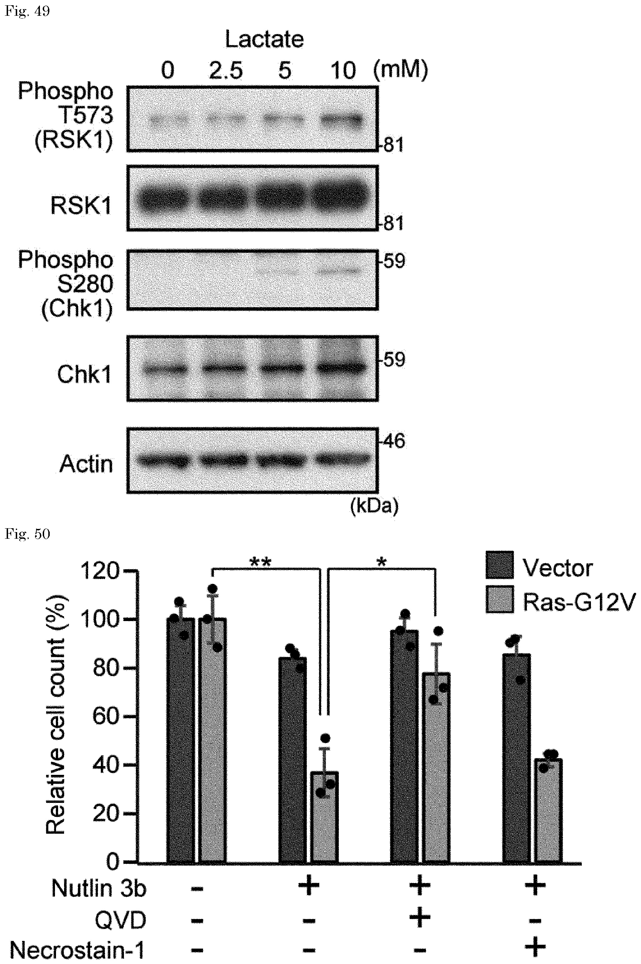

In order to explore the physiological significance of enhanced glycolysis in senescence, a conditioned medium consisting of senescent cells and non-senescent cells was prepared and added to human fibroblasts at the early passage. PGAM-Chk1 interaction was potentiated by culture supernatant from the senescent cells, but not potentiated in the control (Fig. 44). It was considered from this that a component potentiating the PGAM-Chkl interaction was included in the culture supernatant from the senescent cells. Lactate was considered to be a candidate for such a component, and human fibroblasts were treated with lactate. As a result, accumulation of HIF-2$\alpha$, phosphorylation of Ser12 thereof, and enhancement of PGAM-Chk1 interaction were observed in a lactate concentration-dependent manner (Fig. 45). This result was consistent with elevated lactate levels in the serum of aged mice and the culture supernatant of senescent cells. In addition, lactate stimulated SASP expression, but did not provide a difference in p21 level from the control (Fig. 46). Furthermore, the cell surface receptor for lactate, GPR81, was upregulated in senescent cells (Fig. 47), but knockdown thereof eliminated the potentiation of PGAM-Chkl interaction by lactate (Fig. 48). GPR81 signaling is known to be partially involved in oncogenic modules including the Ras pathway (Ohno et al., 2018). In senescent cells or lactate-treated cells, it was observed that RSK1 kinase, which is downstream of the Ras pathway, was activated, accompanied by phosphorylation of Ser280 of Chkl (Li et al., 2012), which is a known target of RSK (Fig. 49).

9. Examination of senolytic effect by suppressing Survivin expression

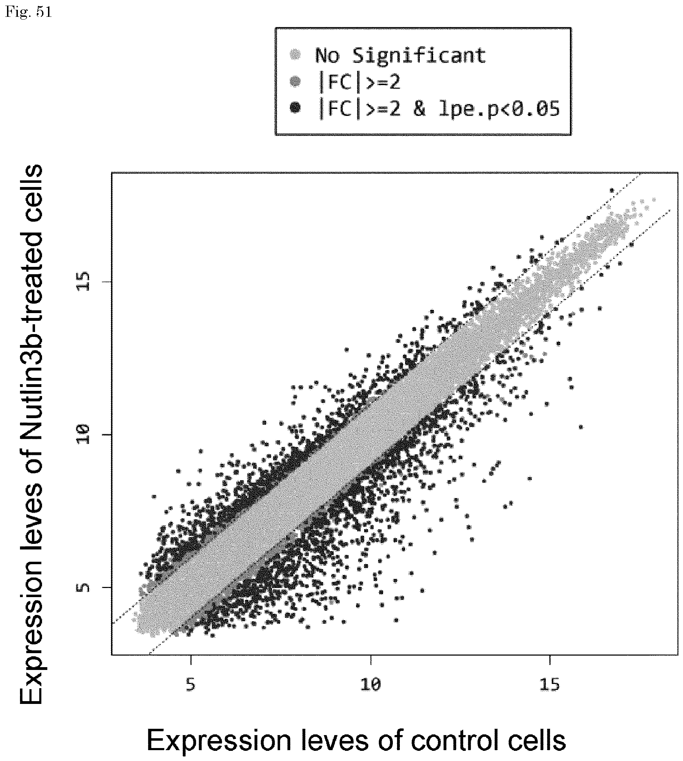

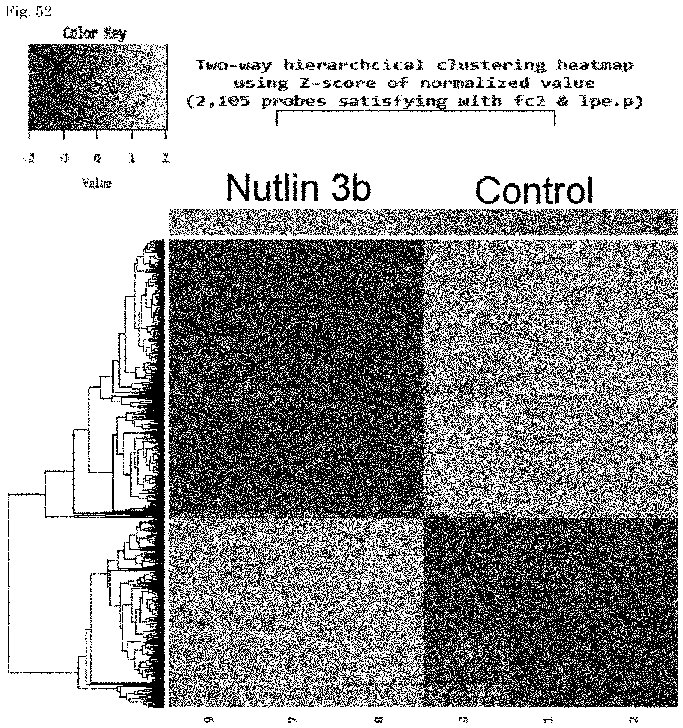

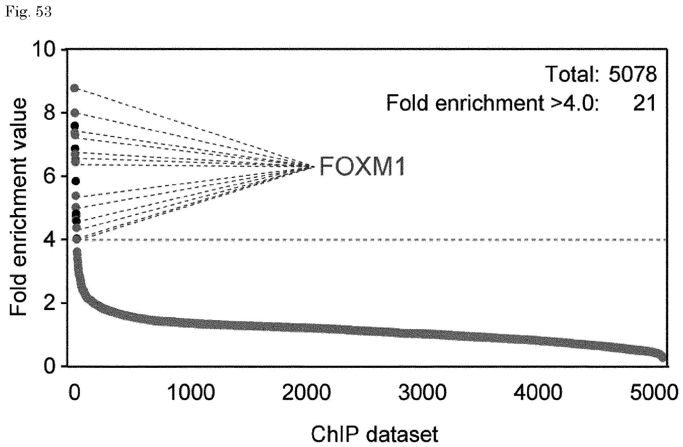

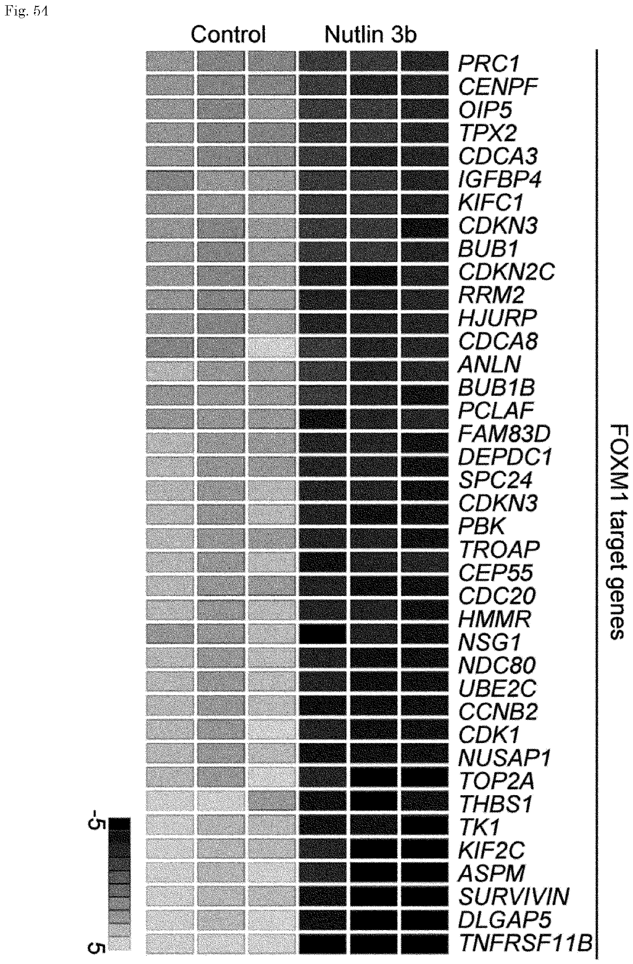

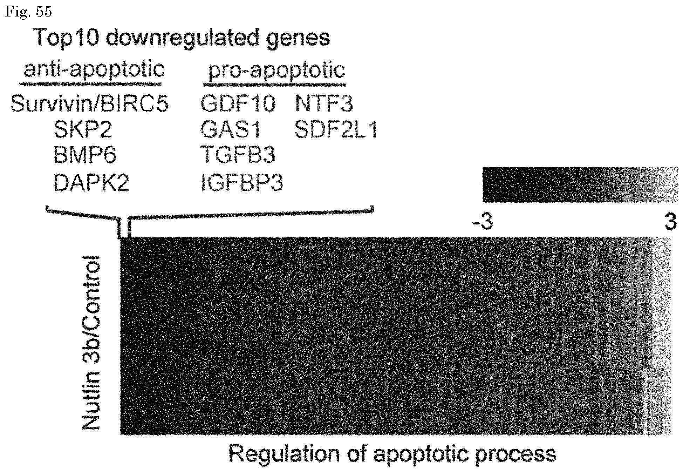

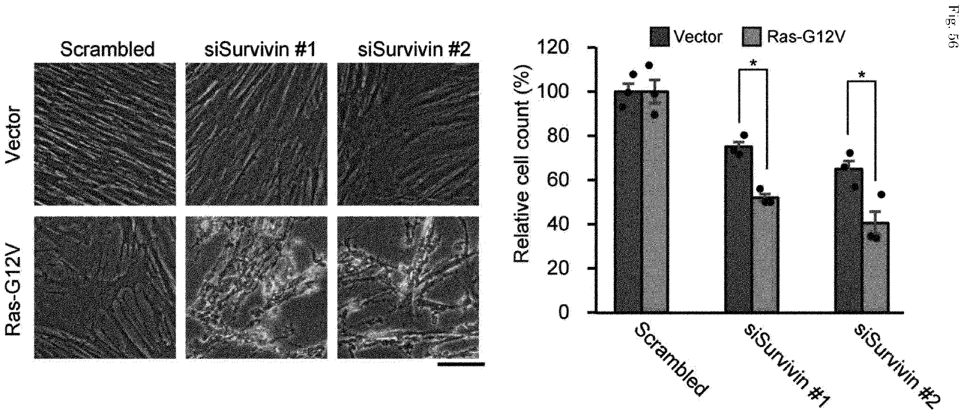

It was observed that the apoptosis inhibitor QVD-Oph restored the viability of senescent cells under nutlin 3b treatment (Fig. 50). In addition, transcriptome analysis was carried out in order to clarify the molecular mechanism of senolysis by inhibition of PGAM-Chkl binding (Fig. 51). 1,168 Genes whose expression was reduced 2-fold or more by nutlin 3b in senescent cells (Fig. 52) and ChIP (chromatin immunoprecipitation) information reported in the National Center for Biotechnology Information (NCBI) were compared and examined by enrichment analysis. The latter information consists of 12,655 ChIP datasets from 903 antigens (transcription factors and epigenetic regulators). As a result, 5,078 ChIP datasets showed 10 or more duplicate genes with the group of genes whose expression was reduced 2-fold or more by nutlin 3b, and 21 datasets showed the highest value (4-fold or more) of fold enrichment (FE). Of these 21 data sets, 12 sets were ChIP data for FOXM1, and the highest enrichment score showed 8.77 (Fig. 53), but several other factors (HDAC2, MYBL2, E2F4, RBL2/p130, Rad21) were also included. In addition, about 26.0% (39 genes) of the top 150 genes controlled 3.45-fold or more by nutlin 3b have been reported as FOXM1 targets (Fig. 54) (Lam et al., 2013).
A microarray dataset on nutlin 3b-treated senescent cells was analyzed for 202 genes (GO:0042981) classified as regulation of the apoptotic process. The top 10 downregulated genes out of the 41 genes affected by nutlin 3b treatment were listed (Fig. 55). Survivin/BIRC5, which is one of the IAP proteins, was most remarkably downregulated (about 30-fold) in the anti-apoptotic family. Consistently, Survivin protein is downregulated in the nutlin 3b-treated senescent cells. In fact, knockdown of survivin in senescent cells induced moderate senolysis to apoptosis (Fig. 56), suggesting that the senolytic effect of nutlin 3b is mediated by suppression of the HIF2-FOXM1-survivin pathway.

10. Examination of Survivin expression controled by FOXM1

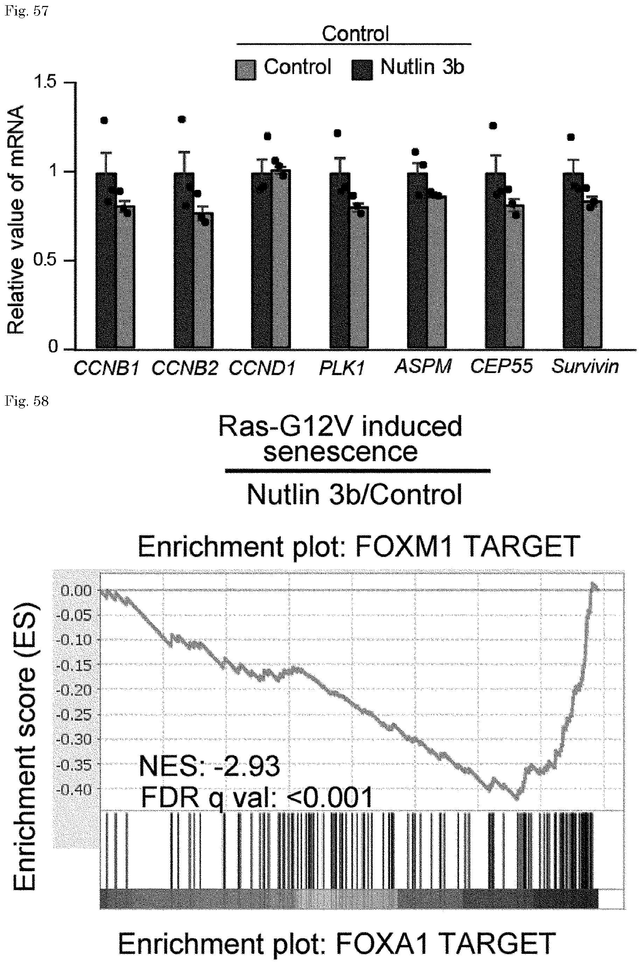

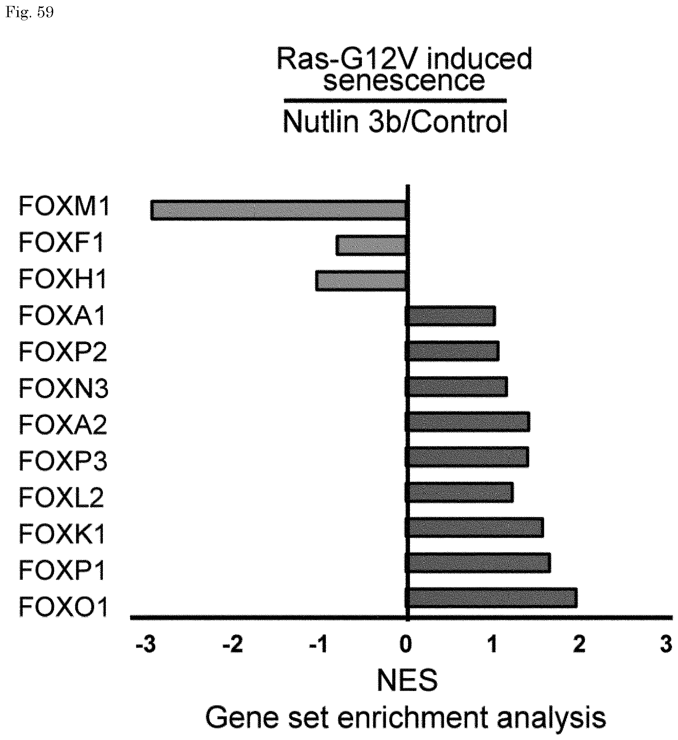

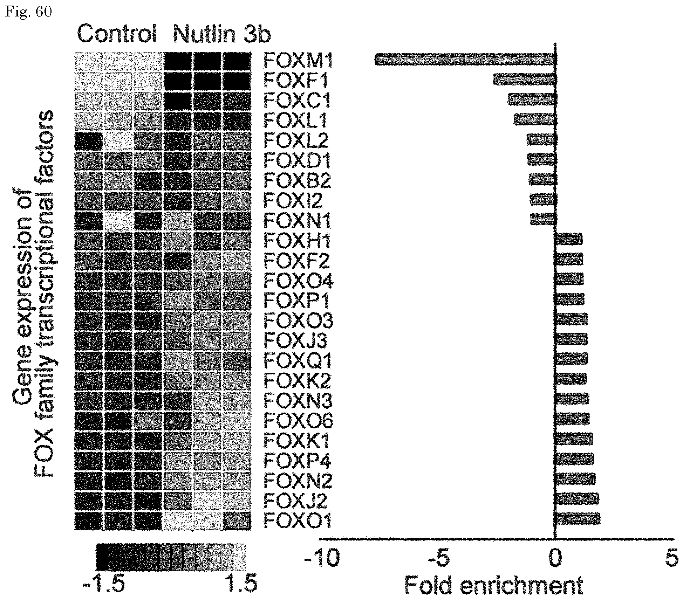

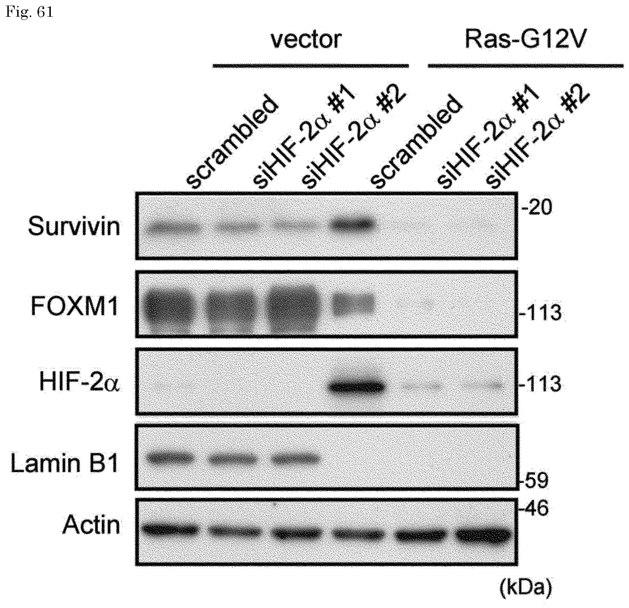

It was found that in addition to Survivin, mRNAs for other transcription targets of the FoxMl gene, including cell cycle regulators (CCNB1, B2, and D1) and mitosis-related genes (PLK1, ASPM, and CEP55) (Lamet et al., 2013) were also significantly affected by nutlin 3b treatment (Fig. 57). GESA (gene set enrichment analysis) of the microarray data showed that the FoxMl target was significantly downregulated (FDRq value of less than 0.001) (Figs. 58 and 59). In the Fox family, FoxO protein is deeply involved in the senescence process. In addition, among the members of the FOX transcription family, the FoxMl mRNA is most dramatically downregulated by nutlin 3b (Fig. 60). Furthermore, inactivation of HIF-$2\alpha$ downregulated FoxMl protein in senescent cells, but not in young passage cells (Fig. 61), which is consistent with the idea that FoxMl is transcriptionally regulated by HIF-$2\alpha$ (Bai et al., 2019).
The primers used in the present Examples are summarized in the tables below (SEQ ID NOs: 11 to 76).

[Table 1-1]

**Table S2. Primers for real-time qRT-PCR**

| Name | Sequence | Sequence ID |
|---|---|---|
| human *Rpl13a* qRT Fw | 5'-CTG GAC CGT CTC AAG GTG TT-3' | 11 |
| human *Rpl13a* qRT Re | 5'-GCC CCA GAT AGG CAA ACT T-3' | 12 |
| human *Aldoa* qRT Fw | 5'-GGT GTC ATC CTC TTC CAT GAG-3' | 13 |
| human *Aldoa* qRT Re | 5'-GTA GTC TCG CCA TTT GTC CC-3' | 14 |
| human *Gapdh* qRT Fw | 5'-CTT TGT CAA GCT CAT TTC CTG G-3' | 15 |
| human *Gapdh* qRT Re | 5'-TCT TCC TCT TGT GCT CTT GC-3' | 16 |
| human *Pgk1* qRT Fw | 5'-GCT TCT GGG AAC AAG GTT AAA G-3' | 17 |
| human *Pgk1* qRT Re | 5'-CTG TGG CAG ATT GAC TCC TAC-3' | 18 |
| human *Pgam1* qRT Fw | 5'-GGA GGC GCT CCT ATG ATG TC-3' | 19 |
| human *Pgam1* qRT Re | 5'-ATC TTC TGT GAG GTC TGC ATA C-3' | 20 |
| human *Eno1* qRT Fw | 5'-TTG GAG CAG AGG TTT ACC AC-3' | 21 |
| human *Eno1* qRT Re | 5'-TTC CCA ATA GCA GTC TTC AGC-3' | 22 |
| human CCNB1 qRT Fw | 5'-TGT GGA TGC AGA AGA TGG AG-3' | 23 |
| human CCNB1 qRT Re | 5'-GTG ACT TCC CGA CCC AGT AG-3' | 24 |
| human CCNB2 qRT Fw | 5'-AGT TCC AGT TCA ACC CAC CAA-3' | 25 |
| human CCNB2 qRT Re | 5'-TTG CAG AGC AAG GCA TCA GA-3' | 26 |
| human CCND1 qRT Fw | 5'-CGT GGC CTC TAA GAT GAA GG-3' | 27 |

(continued)

**Table S2. Primers for real-time qRT-PCR**

| Name | Sequence | Sequence ID |
|---|---|---|
| human CCND1 qRT Re | 5'-CCA CTT GAG CTT GTT CAC CA-3' | 28 |
| human PLK1 qRT Fw | 5'-CTG ACC ATT CCA CCA AGG TT-3' | 29 |
| human PLK1 qRT Re | 5'-AGT CGA CCA CCT CAC CTG TC-3' | 30 |
| human ASPM qRT Fw | 5'-GAG ACC TTG GTG GAA TAC CTG C-3' | 31 |
| human ASPM qRT Re | 5'-ACG AAG ATC CAA AAG CCT TGC AC-3' | 32 |
| human CEP55 qRT Fw | 5'-TCG ACC GTC AAC ATG TGC AGC A-3' | 33 |
| human CEP55 qRT Re | 5'-GGC TCT GTG ATG GCA AAC TCA TG-3' | 34 |
| human BIRC5 qRT Fw | 5'-CCA CTG AGA ACG AGC CAG AC-3' | 35 |
| human BIRC5 qRT Re | 5'-GAC AGA AAG GAA AGC GCA AC-3' | 36 |
| human GPR81 qRT Fw | 5'-CTG GTC ATC CTG GGA ACA GT-3' | 37 |
| human GPR81 qRT Re | 5'-CTT CTT CAT CCG AGC CTG TC-3' | 38 |
| human BIM qRT Fw | 5'-TCC CTG CTG TCT CGA TCC TC-3' | 39 |
| human BIM qRT Re | 5'-GGT CTT CGG CTG CTT GGT AA-3' | 40 |
| human PUMA qRT Fw | 5'-GAC GAC CTC AAC GCA CAG TA-3' | 41 |
| human PUMA qRT Re | 5'-AGG AGT CCC ATG ATG AGA TTG T-3' | 42 |
| human BAK qRT Fw | 5'-GTT TTC CGC AGC TAC GTT TTT-3' | 43 |
| human BAK qRT Re | 5'-GCA GAG GTA AGG TGA CCA TCT C-3' | 44 |

[Table 1-2]

**Table S2. Primers for real-time qRT-PCR (continued)**

| Name | Sequence | Sequence ID |
|---|---|---|
| human BAD qRT Fw | 5'-GCA CAG CAA CGC AGA TGC-3' | 45 |
| human BAD qRT Re | 5'-AAG TTC CGA TCC CAC CAG G-3' | 46 |
| human BAX qRT Fw | 5'-TCT ACT TTG CCA GCA AAC TGG TGC-3' | 47 |
| human BAX qRT Re | 5'-TGT CCA GCC CAT GAT GGT TCT GAT-3' | 48 |
| human BIK qRT Fw | 5'-AGA CCC TCC TGT ATG AGC AG-3' | 49 |
| human BIK qRT Re | 5'-GCA TTC CAA AGA ATC GAA GTC CT-3' | 50 |
| human BMF qRT Fw | 5'-GAG CCA TCT CAG TGT GTG GAG-3' | 51 |
| human BMF qRT Re | 5'-GCC AGC ATT GCC ATA AAA GAG TC-3' | 52 |
| human HRK qRT Fw | 5'-TGC TCG GCA GGC GGA ACT TGT AG-3' | 53 |
| human HRK qRT Re | 5'-CTT TCT CCA GGA CAC AGG G-3' | 54 |
| human BID qRT Fw | 5'-CTA CGA TGA GCT GCA GAC TG-3' | 55 |
| human BID qRT Re | 5'-GAT GCT ACG GTC ATG CTG TC-3' | 56 |
| human NOXA qRT Fw | 5'-CCT GAG CAG AAG AGT TTG GA-3' | 57 |
| human NOXA qRT Re | 5'-GCT GGA AGT CGA GTG TGC TA-3' | 58 |
| human p21 qRT Fw | 5'-AGT CAG TTC CTT GTG GAG CC-3' | 59 |
| human p21 qRT Re | 5'-CAT GGG TTC TGA CGG ACA T-3' | 60 |
| human IL6 qRT Fw | 5'-TGA GAG TAG TGA GGA ACA AG-3' | 61 |
| human IL6 qRT Re | 5'-CGC AGA ATG AGA TGA GTT G-3' | 62 |
| human TNFα qRT Fw | 5'-TTG CCT TGA CCC TGAAGC TC-3' | 63 |
| human TNFα qRT Re | 5'-TCA GAA GCC AGC GTT CAC C-3 | 64 |
| human CSF2 qRT Fw | 5'-ACT ACA AGCA GC ACT GCC CT-3' | 65 |
| human CSF2 qRT Re | 5'-AGC AGT CAA AGG GGATGA CA-3' | 66 |
| mouse p16Ink4 qRT Fw | 5'-CCC AAC GCC CCG AAC T-3' | 67 |
| mouse p16Ink4 qRT Re | 5'-GCA GAA GAG CTG CTA CGT GAA-3' | 68 |
| mouse IL6 qRT Fw | 5'-GAT AAG CTG GAG TCA CAG AAG G-3' | 69 |
| mouse IL6 qRT Re | 5'-GGA ATG TCC ACA AAC TGA TAT GC-3' | 70 |

## EP 4 400 117 A1

(continued)

**Table S2. Primers for real-time qRT-PCR (continued)**

| Name | Sequence | Sequence ID |
| --- | --- | --- |
| mouse CXCL1 qRT Fw | 5'-TTG CCT TGA CCC TGA AGC TC-3' | 71 |
| mouse CXCL1 qRT Re | 5'-TCA GAA GCC AGC GTT CAC C-3' | 72 |
| mouse TNFα qRT Fw | 5'-CCA AGG TCAACC TCC TCT CTG-3' | 73 |
| mouse TNFα qRT Re | 5'-CCA AAG TAG ACC TGC CCA GAC-3' | 74 |
| mouse CCL5 qRT Fw | 5'-GGG TAC CAT GAA GAT CTC TGC-3' | 75 |
| mouse CCL5 qRT Re | 5'-TCT AGG GAG AGG TAG GCA AAG-3' | 76 |

INDUSTRIAL APPLICABILITY

The method for screening a senolytic drug according to the present invention can remarkably improve the efficiency of senolytic drug screening by carrying out primary screening including a step of measuring inhibitory activity of binding between PGAM and Chk1 before a step of measuring selective senolytic activity. The present inventors have found that the binding between PGAM and Chkl was not observed in young normal cells, but was remarkably enhanced in senescent cells, thereby causing the occurrence of enhanced glycolytic metabolism. Accordingly, by first screening many substances for a substance having inhibitory activity on binding between PGAM and Chk1, a substance that can inhibit the binding between PGAM and Chkl in senescent cells can be easily obtained, and by measuring the selective senolytic activity thereof, a senolytic drug can be efficiently found. In fact, the screening method of the present invention was used to narrow down a library of more than 2300 existing drugs having established safety to about 200 candidate drugs by primary screening, and a selective senolytic activity test was carried out to finally succeed in identifying about 40 novel senolytic drugs. Research by the present inventors has also confirmed that even in stress-induced cellular senescence, which is telomere-independent cellular senescence, glycolytic metabolism enhancement due to the binding between PGAM and Chkl occurs, as in so-called senescent cells, and according to the screening method of the present invention, it is also possible to find a substance that exhibits a therapeutic effect not only on an age-related disease but also on a disease developed by stress-induced cellular senescence.

Sequence listing

**Claims**

1. A senolytic drug that inhibits binding between PGAM and Chk1 and selectively kills senescent cells.

2. The senolytic drug according to claim 1, wherein the senolytic drug comprises, as an active ingredient, at least one selected from the group consisting of an RSK1 kinase inhibitor, an Fak kinase inhibitor, an inhibitor of a signal pathway involving Fak kinase, a CDK inhibitor, a calcium antagonist, a cardiac glycoside, a DNA-damaging agent, an antimicrobial agent, an Aurora kinase inhibitor, a flavonoid, a PI3K kinase inhibitor, an HDAC inhibitor, a therapeutic agent for age-related macular degeneration, a p38 MAPK inhibitor, an mTOR inhibitor, a tyrosine kinase inhibitor, a Bcl-2 inhibitor, a statin, a serotonin receptor antagonist, a further kinase inhibitor, and nutlin-3b.

3. The senolytic drug according to claim 2, wherein

the RSK1 kinase inhibitor is an RSK1 antisense nucleic acid, an RSK1 siRNA, or BID1870;
the Fak kinase inhibitor is PF-00562271 or PF-431396;
the inhibitor of a signal pathway involving Fak kinase is PHA-665752 or CP-100356;
the CDK inhibitor is SNS-032, AZD5438, Flavopiridol (Alvocidib), PHA-793887, AT7519, or PHA-767491;
the calcium antagonist is Manidipine or Lomerizine 2HCl;
the cardiac glycoside is Proscillaridin A or Digoxin;
the DNA-damaging agent is Doxorubicin (Adriamycin) HCl, Daunorubicin HCl, Epirubicin HCl, Mitoxantrone 2HCl, or Hydroxy Camptothecine;
the antimicrobial agent is Aminoacridine, Triclosan, or Ethacridine lactate;
the Aurora kinase inhibitor is AMG-900 or JNJ-7706621;
the flavonoid is Chrysin;

the PI3K kinase inhibitor is PF-05212384 or XL147;
the HDAC inhibitor is Trichostatin A (TSA);
the therapeutic agent for age-related macular degeneration is Verteporfin;
the p38 MAPK inhibitor is SB203580 or Doramapimod (BIRB796);
the mTOR inhibitor is AZD8055 or KU-0063794;
the tyrosine kinase inhibitor is Neratinib, Vargatef, or NVP-BHG712;
the Bcl-2 inhibitor is TW-37;
the statin is Mevastatin;
the serotonin receptor antagonist is RS-127445; and
the further kinase inhibitor is BMS-345541 or CX-4945.

4. A method for screening a senolytic drug, comprising:

(A) a step of measuring inhibitory activity of a test substance on binding between PGAM and Chk1; and
(B) a step of measuring selective senolytic activity of a test substance determined to have inhibitory activity on binding between PGAM and ChkI in step (A).

5. The method according to claim 4, wherein step (B) comprises measuring toxic activity of a test substance on young cells and toxic activity thereof on senescent cells, and selecting a test substance having no toxic activity on young cells and having toxic activity on senescent cells.

6. A senolytic drug that has an action of inactivating HIF-2$\alpha$ and selectively kills senescent cells.

7. The senolytic drug according to claim 6, wherein the senolytic drug comprises an HIF-2$\alpha$ inhibitor as an active ingredient.

8. The senolytic drug according to claim 7, wherein the HIF-2$\alpha$ inhibitor is an antisense nucleic acid or siRNA of HIF-2$\alpha$.

9. A method for screening a senolytic drug, comprising:

(X) a step of measuring HIF-2$\alpha$ inhibitory activity of a test substance; and
(Y) a step of measuring selective senolytic activity of a test substance determined to have HIF-2$\alpha$ inhibitory activity in step (X).

10. The method according to claim 9, wherein step (Y) comprises measuring toxic activity of a test substance on young cells and toxic activity thereof on senescent cells, and selecting a test substance having no toxic activity on young cells and having toxic activity on senescent cells.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14-1

Fig. 14-2

Fig. 15

Fig. 16

## Nanobit assay:

Fig. 17

Fig. 18

Fig. 19

Fig. 20

Fig. 21

Fig. 22

Fig. 23

Fig. 24

Fig. 25

**BIM**

Relative value of mRNA

Nut3b  **-**  **+**

Senescent cells

Fig. 26

**BIM**

Relative value of mRNA

siHIF2α  **-**  **+**

Senescent cells

Fig. 27

Fig. 28

Fig. 29

Fig. 30

Fig. 31-1

Fig. 31-2

**Drug 5μM**

Fig. 32

## Senolytic candidate drugs

Nutlin 3b

**RSK90 inhibitor**
BI D1870

**Screening hit compounds(41)**

**Group 1. FAK, Mdr, cMET inhibitors (4)**
PHA-665752 (2.56)
CP-100356 (2.38)
PF-00562271 (1.93)
PF-431396 (1.37)

**Group 2. CDK inhibitors (6)**
SNS-032 (1.65)
AZD5438 (1.54)
Flavopiridol (1.51)
PHA-793887 (1.32)
AT7519 (1.25)
PHA-767491 (1.14)

**Group 3. Calcium antagonists (2)**
Manidipine (1.91)
Lomerizine (1.26)

**Group 4. Cardiac glycosides (2)**
Proscillaridin A (1.51)
Digoxin (1.32)

**Group 5. DNA-damaging drugs (5)**
Doxorubicin (1.46)
Daunorubicin (1.33)
Epirubicin (1.39)
Mitoxantone (1.52)
Camptothecine (1.18)

**Group 6. Antimicrobial drugs (3)**
Triclazan (1.26)
Aminacrine (1.47)
Ethacridine (1.22)

**Group 7. Aurora kinase inhibition (2)**
JNJ-7706621 (1.35)
AMG900 (1.40)

**Others**

**Flavone or PI3K inhibition (3)**
Chrysin (1.22)
PF-05212384 (1.17)
XL147 (1.21)

**HDAC inhibition (1)**
Trichostatin A (1.70)

**Therapeutic drug for age-related macula lutea (1)**
Verteporfin (2.08)

**p38 kinase inhibition (1)**
SB203580 (1.24)
BIRB 796 (1.19)

**TOR inhibitors (2)**
AZD8055 (1.23)
KU-0063794 (1.16)

**Tyrosine kinase inhibition (3)**
Neratinib (1.39)
Vargatef (1.28)
NVP-BHG712 (1.33)

**Bcl-2 inhibition (1)**
TW-37 (1.14)

**Statin (1)**
Mevastatin (1.21)

**Serotonin receptor antagonist (1)**
RS-127445 (1.21)

**Other kinase inhibition (2)**
BMS-34541 (1.22)
CX-4945 (1.19)

Fig. 33

Fig. 34

Fig. 35

Fig. 36A

Fig. 36B

Fig. 37

Fig. 38

Ras-G12V induced
senescence

MG132          −    −    +

Nutlin3b       −    +    +

HIF-2α

-113

Actin

-46

(kDa)

Fig. 39

*HIF-2α*

Relative value of mRNA

1.5

1

0.5

0

Nutlin3b       −    +    +
MG132          −    −    +

Fig. 40

|  |  | 12 | 19 |  |
|---|---|---|---|---|
| Hs_HIF-2α | 1- MTADKEK | KRSSS | ERKEKS | RDAARCRRSKE - 30 |
| Mm_HIF-2α | 1- MTADKEK | KRSSS | ELRKEKS | RDAARCRRSKE - 30 |
| Rn_HIF-2α | 1- MTADKEK | KRSSS | ELRKEKS | RDAARCRRSKE - 30 |
| Gg_HIF-2α | 1- MTADKEK | KRSSS | ERKEKS | RDAARCRRSKE - 30 |
| Xt_HIF-2α | 1- MTAEKEK | KRNSS | ERKEKS | RDAARCRRSKE - 30 |

Motif 1    Motif 2

HIF-2α_S12A    -----------A-----------------

HIF-2α_S19A    -----------------A-----------

Chk1 consensus motif    R/K-R/K-x-x-S/T

Fig. 41

Fig. 42

Fig. 43

Fig. 44

Fig. 45

Fig. 46

Fig. 47

GPR81

Fig. 48

Fig. 49

Fig. 50

Fig. 51

Fig. 52

Fig. 53

Fig. 54

Fig. 55

## Top10 downregulated genes

anti-apoptotic        pro-apoptotic

Survivin/BIRC5    GDF10   NTF3
     SKP2         GAS1    SDF2L1
     BMP6         TGFB3
     DAPK2        IGFBP3

-3                                      3

Nutlin 3b/Control

Regulation of apoptotic process

Fig. 56

Fig. 57

Fig. 58

Ras-G12V induced
senescence

Nutlin 3b/Control

Enrichment plot: FOXM1 TARGET

Enrichment plot: FOXA1 TARGET

Fig. 59

Ras-G12V induced senescence / Nutlin 3b/Control

NES

Gene set enrichment analysis

Fig. 60

Fig. 61

## INTERNATIONAL SEARCH REPORT

| International application No. |
| --- |
| **PCT/JP2022/033445** |

### A. CLASSIFICATION OF SUBJECT MATTER

*A61K 45/00*(2006.01)i; *A61K 31/08*(2006.01)i; *A61K 31/138*(2006.01)i; *A61K 31/166*(2006.01)i; *A61K 31/352*(2006.01)i;
*A61K 31/366*(2006.01)i; *A61K 31/4025*(2006.01)i; *A61K 31/403*(2006.01)i; *A61K 31/407*(2006.01)i;
*A61K 31/409*(2006.01)i; *A61K 31/4155*(2006.01)i; *A61K 31/4178*(2006.01)i; *A61K 31/4188*(2006.01)i;
*A61K 31/4196*(2006.01)i; *A61K 31/437*(2006.01)i; *A61K 31/4375*(2006.01)i; *A61K 31/438*(2006.01)i;
*A61K 31/444*(2006.01)i; *A61K 31/4709*(2006.01)i; *A61K 31/473*(2006.01)i; *A61K 31/495*(2006.01)i;
*A61K 31/498*(2006.01)i; *A61K 31/4985*(2006.01)i; *A61K 31/502*(2006.01)i; *A61K 31/505*(2006.01)i;
*A61K 31/506*(2006.01)i; *A61K 31/519*(2006.01)i; *A61K 31/52*(2006.01)i; *A61K 31/53*(2006.01)i; *A61K 31/7012*(2006.01)i;
*A61K 31/7028*(2006.01)i; *A61K 31/704*(2006.01)i; *A61K 31/7088*(2006.01)i; *A61K 31/713*(2006.01)i;
*A61P 43/00*(2006.01)i; *C12Q 1/02*(2006.01)i; *G01N 33/15*(2006.01)i; *G01N 33/52*(2006.01)i

FI: A61K45/00 ZNA; A61K31/08; A61K31/138; A61K31/166; A61K31/352; A61K31/366; A61K31/4025; A61K31/403;
A61K31/407; A61K31/409; A61K31/4155; A61K31/4178; A61K31/4188; A61K31/4196; A61K31/437; A61K31/4375;
A61K31/438; A61K31/444; A61K31/4709; A61K31/473; A61K31/495; A61K31/498; A61K31/4985; A61K31/502;
A61K31/505; A61K31/506; A61K31/519; A61K31/52; A61K31/53; A61K31/7012; A61K31/7028; A61K31/704;
A61K31/7088; A61K31/713; A61P43/00 105; C12Q1/02; G01N33/15 Z; G01N33/52 Z

According to International Patent Classification (IPC) or to both national classification and IPC

### B. FIELDS SEARCHED

Minimum documentation searched (classification system followed by classification symbols)

A61K45/00; A61K31/08; A61K31/138; A61K31/166; A61K31/352; A61K31/366; A61K31/4025; A61K31/403; A61K31/407;
A61K31/409; A61K31/4155; A61K31/4178; A61K31/4188; A61K31/4196; A61K31/437; A61K31/4375; A61K31/438;
A61K31/444; A61K31/4709; A61K31/473; A61K31/495; A61K31/498; A61K31/4985; A61K31/502; A61K31/505;
A61K31/506; A61K31/519; A61K31/52; A61K31/53; A61K31/7012; A61K31/7028; A61K31/704; A61K31/7088;
A61K31/713; A61P43/00; C12Q1/02; G01N33/15; G01N33/52

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

| ☑ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **25 October 2022** | **01 November 2022** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
|---|
| **PCT/JP2022/033445** |

### C. DOCUMENTS CONSIDERED TO BE RELEVANT

| Category* | Citation of document, with indication, where appropriate, of the relevant passages | Relevant to claim No. |
|---|---|---|
| X | TRIANA-MARTINEZ, F. et al. Identification and characterization of Cardiac Glycosides as senolytic compounds. Nat. Commun. 2019, vol. 10, 4731, pp. 1-12 <br> in particular, see abstract, results, fig. 1, 5 | 1-3 |
| A | | 4-10 |
| X | JP 2020-521734 A (UNITED KINGDOM RESEARCH AND INNOVATION) 27 July 2020 (2020-07-27) <br> claims, examples | 1-3 |
| A | | 4-10 |
| X | WO 2019/133904 A1 (UNITY BIOTECHNOLOGY, INC.) 04 July 2019 (2019-07-04) <br> claims, fig. 1D | 1-3 |
| A | | 4-10 |
| X | WO 2019/104065 A1 (TURRINII PHARMACEUTICAL, LLC) 31 May 2019 (2019-05-31) <br> claims, paragraph [0130] | 1-3 |
| A | | 4-10 |
| X | WO 2017/008060 A1 (UNITY BIOTECHNOLOGY, INC.) 12 January 2017 (2017-01-12) <br> claims, pp. 26, 27, 31, 34 | 1-3 |
| A | | 4-10 |
| X | 三河隆太ほか, 老化細胞除去モデルとsenolytic薬, アンチ・エイジング医学ー日本抗加齢医学会雑誌, 2017, vol. 13, no. 4, pp. 486-492 <br> in particular, see pp. 490-491, table 2, (MIKAWA, Ryuta et al. Semigenetic and pharmacological approaches to senolysis. Anti-aging medicine.) | 1, 2 |
| A | | 3-10 |
| X | JP 2018-516595 A (ARROWHEAD PHARMACEUTICALS INC) 28 June 2018 (2018-06-28) <br> claims, examples | 6 |
| A | | 7-10 |
| A | MIKAWA, T. et al. Phosphoglycerate Mutase Cooperates with Chk1 Kinase to Regulate Glycolysis. iScience. 2020, vol. 23, issue 7, 101306 , pp. 1-16 <br> in particular, see abstract, results | 1-10 |
| A | JP 2018-194299 A (UNIV KYOTO) 06 December 2018 (2018-12-06) <br> claims, examples | 1-10 |

Form PCT/ISA/210 (second sheet) (January 2015)

**INTERNATIONAL SEARCH REPORT**

| International application No. |
| --- |
| **PCT/JP2022/033445** |

| Box No. I Nucleotide and/or amino acid sequence(s) (Continuation of item 1.c of the first sheet) |
| --- |

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    The "form of an Annex C/ST.25 text file" is replaced with the "form of ST.26."

Form PCT/ISA/210 (continuation of first sheet) (January 2015)

| Patent document cited in search report | | | Publication date (day/month/year) | Patent family member(s) | | | Publication date (day/month/year) |
|---|---|---|---|---|---|---|---|
| JP | 2020-521734 | A | 27 July 2020 | WO | 2018/215795 | A2 | |
| | | | | claims, examples | | | |
| | | | | US | 2020/0121620 | A1 | |
| | | | | EP | 3630125 | A2 | |
| WO | 2019/133904 | A1 | 04 July 2019 | US | 2019/0248837 | A1 | |
| | | | | EP | 3548504 | A1 | |
| WO | 2019/104065 | A1 | 31 May 2019 | (Family: none) | | | |
| WO | 2017/008060 | A1 | 12 January 2017 | US | 2018/0193458 | A1 | |
| JP | 2018-516595 | A | 28 June 2018 | WO | 2016/196239 | A1 | |
| | | | | claims, examples | | | |
| | | | | US | 2016/0272970 | A1 | |
| | | | | EP | 3270903 | A1 | |
| JP | 2018-194299 | A | 06 December 2018 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018194299 A

### Non-patent literature cited in the description

- **WARBURG O.** *Science,* 1956, vol. 124, 269-270
- **ADLER et al.** *Genes Dev.,* 2007, vol. 21, 3244-3257
- **COPPE et al.** *PLoS Biol.,* 2008, vol. 6, 2853-2868
- **BAKER et al.** *Nature,* 2011, vol. 479, 232-236
- **CHANG et al.** *Nat. Med.,* 2016, vol. 22, 78-83
- **TSE et al.** *Cancer Res.,* 2008, vol. 68, 3421-3428
- **CHILDS et al.** *Science,* 2016, vol. 354, 472-477
- **ZHU et al.** *Aging Cell,* 2015, vol. 14, 644-658
- **GUERRERO et al.** *Nat Metab.,* 2019, vol. 1, 1074-1088
- **TRIANA-MARTINEZ et al.** *Nat. Commun.,* 2019, vol. 10, 4731
- **KONDOH et al.** *Cancer Res.,* 2005, vol. 65, 177-185
- **MIKAWA et al.** *J. Cell Biol.,* 2014, vol. 204, 729-745
- *CHEMICAL ABSTRACTS,* 501437-28-1
- *CHEMICAL ABSTRACTS,* 939791-41-0
- *CHEMICAL ABSTRACTS,* 717906-29-1
- *CHEMICAL ABSTRACTS,* 477575-56-7
- *CHEMICAL ABSTRACTS,* 142715-48-8
- *CHEMICAL ABSTRACTS,* 345627-80-7
- *CHEMICAL ABSTRACTS,* 602306-29-6
- *CHEMICAL ABSTRACTS,* 146426-40-6
- *CHEMICAL ABSTRACTS,* 718630-59-2
- *CHEMICAL ABSTRACTS,* 844442-38-2
- *CHEMICAL ABSTRACTS,* 942425-68-5
- *CHEMICAL ABSTRACTS,* 89226-50-6
- *CHEMICAL ABSTRACTS,* 101477-54-7
- *CHEMICAL ABSTRACTS,* 466-06-8
- *CHEMICAL ABSTRACTS,* 20830-75-5
- *CHEMICAL ABSTRACTS,* 25316-40-9
- *CHEMICAL ABSTRACTS,* 23541-50-6
- *CHEMICAL ABSTRACTS,* 56390-09-1
- *CHEMICAL ABSTRACTS,* 70476-82-3
- *CHEMICAL ABSTRACTS,* 64439-81-2
- *CHEMICAL ABSTRACTS,* 90-45-9
- *CHEMICAL ABSTRACTS,* 3380-34-5
- *CHEMICAL ABSTRACTS,* 1837-57-6
- *CHEMICAL ABSTRACTS,* 945595-80-2
- *CHEMICAL ABSTRACTS,* 443797-96-4
- *CHEMICAL ABSTRACTS,* 675576-97-3
- *CHEMICAL ABSTRACTS,* 480-40-0
- *CHEMICAL ABSTRACTS,* 1197160-78-3
- *CHEMICAL ABSTRACTS,* 934526-89-3
- *CHEMICAL ABSTRACTS,* 58880-19-6
- *CHEMICAL ABSTRACTS,* 129497-78-5
- *CHEMICAL ABSTRACTS,* 152121-47-6
- *CHEMICAL ABSTRACTS,* 285983-48-4
- *CHEMICAL ABSTRACTS,* 1009298-09-2
- *CHEMICAL ABSTRACTS,* 938440-64-3
- *CHEMICAL ABSTRACTS,* 698387-09-6
- *CHEMICAL ABSTRACTS,* 656247-17-5
- *CHEMICAL ABSTRACTS,* 940310-85-0
- *CHEMICAL ABSTRACTS,* 877877-35-5
- *CHEMICAL ABSTRACTS,* 73573-88-3
- *CHEMICAL ABSTRACTS,* 199864-87-4
- *CHEMICAL ABSTRACTS,* 445430-58-0
- *CHEMICAL ABSTRACTS,* 1009820-21-6
- **IYER et al.** *Genes and Development,* 1998, vol. 12, 149-62
- **SERRANO et al.** *Cell,* 1997, vol. 88 (5), 593-602
- **VASSILEV et al.** *Science,* 2004, vol. 303, 844-848
- **CHANG et al.** *Nat. Med.,* 2016
- **MIKAWA et al.** *iScience,* 2020, vol. 23, 101306
- **LI et al.** *Mol Biol Cell,* 2012, vol. 23, 1582-1592